# EUROPÄISCHE PATENTANMELDUNG

(11) **EP 3 026 118 A1**
(43) Veröffentlichungstag der Anmeldung: **01.06.2016**
(21) Anmeldenummer: 14194668.1
(22) Anmeldetag: 25.11.2014
(51) Int. Cl.: C12P 7/04, C12P 7/24, C12P 7/26, C12N 9/02

(54) **Enzymatisches Verfahren zur Herstellung von 3-Butenal und 3-Buten-1-ol**

(71) Anmelder: LANXESS Deutschland GmbH, 50569 Köln (DE)
(72) Erfinder: Eggert, Thorsten, 45529 Hattingen (DE); Leggewie, Christian, 45481 Mülheim a.d.R. (DE); Weckbecker, Andrea, 50937 Köln (DE); Büttner, Verena, 50181 Bedburg (DE); Dreisbach, Claus, 42799 Leichlingen (DE); Adler, Liv, 51067 Köln (DE)
(74) Vertreter: Godemeyer Blum Lenze Patentanwälte Partnerschaft mbB - werkpatent

(57) **Zusammenfassung**

Die Erfindung betrifft ein Verfahren zur Umsetzung einer Verbindung mit der Formel (II) [Vinylacetyl-CoA, 3-Butenoyl-CoA] 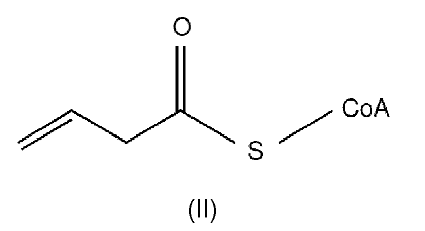 durch Kontaktieren der Verbindung mit der Formel (II) mit einer Aldehyd-Dehydrogenase-Aktivität, wobei eine Verbindung mit der Formel (III) [Vinylacetaldehyd, 3-Butenal] erhalten wird 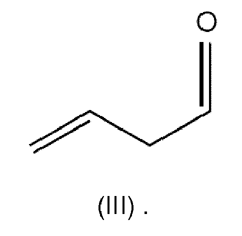 Die Erfindung betrifft weiterhin ein Verfahren zur enzymatischen Herstellung einer Verbindung mit der Formel (IV) [Vinylethanol, 3-Buten-1-ol] 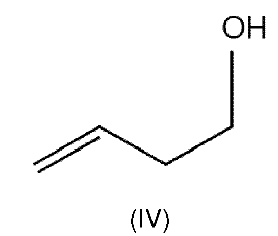 enthaltend die Schritte:
(a) Umsetzung einer Verbindung mit der Formel (II) [Vinylacetyl-CoA, 3-Butenoyl-CoA] 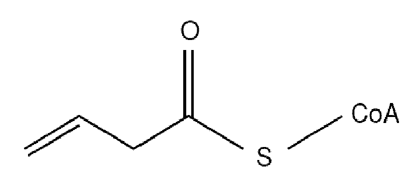 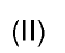 in Anwesenheit einer Aldehyd-Dehydrogenase-Aktivität, wobei eine Verbindung mit der Formel (III) [Vinylacetaldehyd, 3-Butenal] erhalten wird. 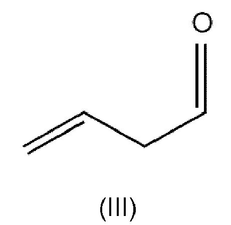 (b) Umsetzung der aus (a) erhaltenen Verbindung mit der Formel (III) in Anwesenheit einer Alkohol-Dehydrogenase-Aktivität, wobei die Verbindung mit der Formel (IV) [Vinylethanol, 3-Buten-1-ol] erhalten wird 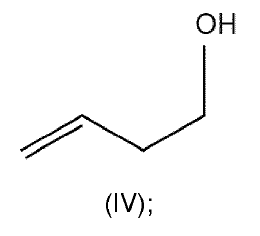 sowie einen Mikroorganismus und eine Zusammensetzung zur Durchführung des Verfahrens.

SEQUENCE LISTING

<110>  Lanxess Deutschland GmbH

<120>  Verfahren zur Herstellung von 3-Butenal und Vinylethanol mit
       Hilfe von Enzymen

<130>  P61009EP

<160>  20

<170>  PatentIn version 3.3

<210>  1
<211>  312
<212>  PRT
<213>  Pseudomonas sp.

<400>  1

Met Asn Gln Lys Leu Lys Val Ala Ile Ile Gly Ser Gly Asn Ile Gly
1               5                   10                  15

Thr Asp Leu Met Ile Lys Val Leu Arg Asn Ala Lys Tyr Leu Glu Met
            20                  25                  30

Gly Ala Met Val Gly Ile Asp Ala Ala Ser Asp Gly Leu Ala Arg Ala
        35                  40                  45

Gln Arg Met Gly Val Thr Thr Thr Tyr Ala Gly Val Glu Gly Leu Ile
    50                  55                  60

Lys Leu Pro Glu Phe Ala Asp Ile Asp Phe Val Phe Asp Ala Thr Ser
65                  70                  75                  80

Ala Ser Ala His Val Gln Asn Glu Ala Leu Leu Arg Gln Ala Lys Pro
                85                  90                  95

Gly Ile Arg Leu Ile Asp Leu Thr Pro Ala Ala Ile Gly Pro Tyr Cys
            100                 105                 110

Val Pro Val Val Asn Leu Glu Glu His Leu Gly Lys Leu Asn Val Asn
        115                 120                 125

Met Val Thr Cys Gly Gly Gln Ala Thr Ile Pro Met Val Ala Ala Val
    130                 135                 140

Ser Arg Val Ala Lys Val His Tyr Ala Glu Ile Val Ala Ser Ile Ser
145                 150                 155                 160

Ser Lys Ser Ala Gly Pro Gly Thr Arg Ala Asn Ile Asp Glu Phe Thr
                165                 170                 175

Glu Thr Thr Ser Lys Ala Ile Glu Val Ile Gly Gly Ala Ala Lys Gly
            180                 185                 190

Lys Ala Ile Ile Ile Met Asn Pro Ala Glu Pro Pro Leu Ile Met Arg
        195                 200                 205

Asp Thr Val Tyr Val Leu Ser Ala Ala Ala Asp Gln Ala Ala Val Ala
    210                 215                 220

Ala Ser Val Ala Glu Met Val Gln Ala Val Gln Ala Tyr Val Pro Gly
225                 230                 235                 240

Tyr Arg Leu Lys Gln Gln Val Gln Phe Asp Val Ile Pro Glu Ser Ala
                245                 250                 255

Pro Leu Asn Ile Pro Gly Leu Gly Arg Phe Ser Gly Leu Lys Thr Ser
            260                 265                 270

Val Phe Leu Glu Val Glu Gly Ala Ala His Tyr Leu Pro Ala Tyr Ala
        275                 280                 285

Gly Asn Leu Asp Ile Met Thr Ser Ala Ala Leu Ala Thr Ala Glu Arg
    290                 295                 300

Met Ala Gln Ser Met Leu Asn Ala
305                 310

<210>  2
<211>  939
<212>  DNA
<213>  Pseudomonas sp.

<400>  2
atgaaccaga aactcaaagt cgcgatcatc ggttcgggca atatcggcac cgacctgatg      60

atcaaggtgc tgcgcaacgc caagtacctg gaaatgggcg ccatggtcgg catcgacgcc     120

gcctccgacg gcctggcccg cgcccagcgc atgggcgtga cgaccaccta tgccggcgtc     180

gaagggctga tcaagctgcc cgaattcgcc gacatcgatt tcgtcttcga cgccacctcg     240

gccagtgccc acgtgcagaa cgaggcgctg ctgcgccagg ccaaacctgg catccgcctg     300

atcgacctga ccccggcggc catcggcccg tactgcgtgc cggtggtcaa tctggaggag     360

cacctcggca agctcaacgt caacatggtt acctgcggcg gccaggcgac catcccgatg     420

gtcgccgcgg tctcccgtgt ggccaaggtc cattacgccg agatcgtcgc ctcgatcagc     480

agcaagtcgg ccggacccgg cacccgcgcc aacatcgacg agttcaccga gaccaccagc     540

aaagccatcg aagtgatcgg tggtgcggcc aagggcaagg cgatcatcat catgaacccg     600

gctgagccgc cgctgatcat gcgcgacacc gtgtatgtgc tgtccgccgc cgccgatcag 660

gccgccgtcg cggcctcggt ggcggaaatg gttcaggcgg tgcaggccta cgtgcccggc 720

tatcgcctga agcagcaggt gcagttcgac gtgatccccg agtccgcgcc gctgaacatc 780

cccggtctcg gccggttcag cgggttgaag acctcggtgt tcctcgaagt cgaaggcgcc 840

gcccattacc tgccggccta cgccggcaac ctcgacatca tgacctccgc cgcgctggct 900

accgccgagc gtatggcgca gtcgatgttg aacgcctga 939

<210> 3
<211> 939
<212> DNA
<213> Artificial

<220>
<223> artificial sequence

<400> 3
atgaaccaga aactgaaagt ggccattatt ggtagcggta atattggcac cgatctgatg 60

attaaagttc tgcgcaatgc caaatatctg gaaatgggtg caatggttgg tattgatgca 120

gcaagtgatg gtctggcacg tgcacagcgt atgggtgtta ccaccaccta tgccggtgtt 180

gaaggtctga ttaaactgcc ggaatttgcc gatattgatt ttgtttttga tgcaaccagc 240

gcaagcgcac atgttcagaa tgaagcactg ctgcgtcagg caaaaccggg tattcgtctg 300

attgatctga caccggcagc aattggtccg tattgtgttc cggttgttaa tctggaagaa 360

catctgggta aactgaatgt gaatatggtt acctgtggtg gtcaggcaac cattccgatg 420

gttgcagcag ttagccgtgt tgcaaaagtt cattatgcag aaattgttgc cagcatcagc 480

agcaaaagcg caggtccggg tacacgtgca aatattgatg aatttaccga aaccacgagc 540

aaagccattg aagttattgg tggtgcagcc aaaggtaaag ccattattat catgaatccg 600

gcagaaccgc ctctgattat gcgtgatacc gtttatgttc tgagcgcagc agcagatcag 660

gcagcagttg cagcaagcgt tgcagaaatg gttcaggcag ttcaggccta tgttccgggt 720

tatcgtctga aacagcaggt tcagtttgat gttattccgg aaagcgcacc gctgaatatt 780

ccgggtctgg gtcgttttag cggtctgaaa accagcgttt ttctggaagt tgaaggtgca 840

gcacattatc tgcctgccta tgcaggtaat ctggatatta tgaccagcgc agcactggca 900

accgcagaac gtatggcaca gagcatgctg aatgcataa 939

<210> 4
<211> 306
<212> PRT
<213> Comamonas testosteroni

<400> 4

Met Lys Lys Ile Lys Cys Ala Leu Ile Gly Pro Gly Asn Ile Gly Thr

1               5                   10                  15

Asp Leu Leu Ala Lys Leu Gln Arg Ser Pro Val Leu Glu Pro Val Trp
            20                  25                  30

Met Val Gly Ile Asp Pro Asp Ser Asp Gly Leu Lys Arg Ala Arg Glu
        35                  40                  45

Met Gly Ile Lys Thr Thr Ala Glu Gly Val Asp Gly Leu Leu Pro His
    50                  55                  60

Met His Ala Asp Gly Val Gln Ile Val Phe Asp Ala Thr Ser Ala Tyr
65                  70                  75                  80

Val His Ala Glu Asn Ser Arg Lys Val Asn Glu Gln Gly Ala Leu Met
                85                  90                  95

Ile Asp Leu Thr Pro Ala Ala Ile Gly Pro Phe Cys Val Pro Pro Val
            100                 105                 110

Asn Leu Met Gln His Leu Gly Ser Gly Ala Met Asn Val Asn Met Val
        115                 120                 125

Thr Cys Gly Gly Gln Ala Thr Ile Pro Met Val Ala Ala Val Ser Arg
    130                 135                 140

Val Gln Ala Val Ala Tyr Gly Glu Ile Val Ala Thr Val Ser Ser Lys
145                 150                 155                 160

Ser Val Gly Pro Gly Thr Arg Lys Asn Ile Asp Glu Phe Thr Arg Thr
                165                 170                 175

Thr Ala Gly Ala Val Glu Lys Val Gly Gly Ala Lys Lys Gly Lys Ala
            180                 185                 190

Ile Ile Val Ile Asn Pro Ala Glu Pro Pro Leu Met Met Arg Asp Thr
        195                 200                 205

Val His Cys Leu Thr Glu Asp Glu Pro Asp Gln Ala Ala Ile Thr Ala
    210                 215                 220

Ser Ile His Gln Met Leu Ala Glu Val Gln Lys Tyr Val Pro Gly Tyr
225                 230                 235                 240

Arg Leu Val Asn Gly Pro Val Phe Asp Gly Lys Arg Val Ser Val Phe
                245                 250                 255

Leu Glu Val Glu Gly Leu Gly Asp Tyr Leu Pro Lys Tyr Ala Gly Asn
            260                 265                 270

Leu Asp Ile Met Thr Ala Ala Ala Ala Arg Thr Ala Glu Met Phe Ala
        275                 280                 285

Glu Gln Met Leu Ala Gly Lys Leu Thr Leu Ala Pro Met Ala Pro Val
    290                 295                 300

Ala Ala
305

<210>  5
<211>  921
<212>  DNA
<213>  Comamonas testosteroni

<400>  5
atgaaaaaaa tcaaatgcgc gctgatcggg ccgggcaata tcggcaccga cctattggcc      60

aagctgcagc gcagccccgt gctggagccg gtgtggatgg tgggcataga ccccgattcg     120

gatggtttga agcgggcgcg tgaaatgggc atcaagacca cggctgaggg cgtggatgga     180

ctgctgccgc atatgcacgc cgatggcgtg caaatcgtgt tcgacgccac cagcgcctat     240

gtgcatgcag agaactcgcg caaggtcaac gagcagggcg cgctgatgat tgacctcacg     300

ccggcggcca taggcccttt ctgcgtgccc ccggtcaacc tgatgcagca cctgggcagc     360

ggcgccatga atgtgaacat ggtcacctgc ggggggcagg ccaccatccc catggtggcg     420

gcggtgagcc gtgttcaggc cgtggcctat ggcgagatcg tggccaccgt gtcctccaag     480

tccgtgggac cgggcacgcg caagaacatt gacgaattca cgcgcaccac ggcaggtgcg     540

gtggagaagg taggcggtgc caagaaaggc aaggccatca tcgtcatcaa cccggccgaa     600

ccccctttga tgatgcgcga caccgtgcac tgcctgaccg aggacgagcc cgaccaggcc     660

gccatcaccg cctccatcca ccagatgctg gccgaggtgc agaaatacgt gcccggttac     720

cgcctggtca acggcccggt gtttgacggc aagcgcgtct ccgtcttcct cgaggtcgaa     780

ggccttggcg actacctgcc caagtacgcg ggcaatctgg acatcatgac cgccgccgcc     840

gcgcgcacgg ccgagatgtt cgccgaacaa atgctggccg gcaagctcac gctcgcaccc     900

atggcccccg tagccgcctg a                                               921

<210>  6
<211>  921
<212>  DNA
<213>  artificial

<220>
<223>  artificial sequence

<400>  6
atgaaaaaaa tcaaatgtgc cctgattggt ccgggtaata ttggcaccga tctgctggca      60

aaactgcaac gtagtccggt tctggaaccg gtttggatgg ttggtattga tccggatagt     120

gatggtctga aacgtgcccg tgaaatgggt attaaaacca cagccgaagg tgtggatggt     180

ctgctgccgc acatgcatgc agatggtgtt cagattgttt ttgatgcaac cagcgcctat     240

gttcatgcag aaaatagccg taaagttaat gaacagggtg cgctgatgat tgatctgaca     300

ccggcagcaa ttggtccgtt ttgtgttccg cctgttaatc tgatgcagca tctgggtagc     360

ggtgcaatga atgttaatat ggttacctgt ggtggtcagg caaccattcc gatggttgca     420

gcagttagcc gtgttcaggc agttgcctat ggtgaaattg ttgcaaccgt tagcagcaaa     480

agcgttggtc ctggcacccg taaaaacatt gatgaattta cccgtaccac cgcaggcgca     540

gttgaaaaag ttggtggtgc aaaaaaaggc aaagccatca ttgttattaa tccggcagaa     600

ccgcctctga tgatgcgtga taccgttcat tgtctgaccg aagatgaacc ggatcaggca     660

gccattaccg caagcattca tcagatgctg gccgaagttc agaaatatgt tccgggttat     720

cgtctggtta atggtccggt gtttgatggt aaacgtgtta gcgtttttct ggaagttgaa     780

ggtctgggtg attatctgcc gaaatatgca ggtaatctgg atattatgac cgcagcagca     840

gcacgtaccg cagaaatgtt tgcagaacaa atgctggcag gtaaactgac cctggctccg     900

atggcaccgg ttgcagccta a                                               921

<210>  7
<211>  877
<212>  PRT
<213>  Clostridium ljungdahlii

<400>  7

Met Lys Val Thr Lys Val Thr Asn Val Glu Glu Leu Met Lys Lys Leu
1               5                   10                  15

Asp Glu Val Thr Ala Ala Gln Lys Lys Phe Ser Ser Tyr Thr Gln Glu
            20                  25                  30

Gln Val Asp Glu Ile Phe Arg Gln Ala Ala Met Ala Ala Asn Ser Ala
        35                  40                  45

Arg Ile Asp Leu Ala Lys Met Ala Val Glu Glu Ser Gly Met Gly Ile
    50                  55                  60

Val Glu Asp Lys Val Ile Lys Asn His Phe Val Ala Glu Tyr Ile Tyr
65                  70                  75                  80

Asn Lys Tyr Lys Gly Glu Lys Thr Cys Gly Val Leu Glu Gln Asp Glu
                85                  90                  95

Gly Phe Gly Met Val Arg Ile Ala Glu Pro Val Gly Val Ile Ala Ala
            100                 105                 110

Val Val Pro Thr Thr Asn Pro Thr Ser Thr Ala Ile Phe Lys Ser Leu
        115                 120                 125

Ile Ala Leu Lys Thr Arg Asn Gly Ile Val Phe Ser Pro His Pro Arg
    130                 135                 140

Ala Lys Lys Ser Thr Ile Ala Ala Ala Lys Ile Val Leu Asp Ala Ala
145                 150                 155                 160

Val Lys Ala Gly Ala Pro Glu Gly Ile Ile Gly Trp Ile Asp Glu Pro
                165                 170                 175

Ser Ile Glu Leu Ser Gln Val Val Met Lys Glu Ala Asp Leu Ile Leu
            180                 185                 190

Ala Thr Gly Gly Pro Gly Met Val Lys Ala Ala Tyr Ser Ser Gly Lys
        195                 200                 205

Pro Ala Ile Gly Val Gly Pro Gly Asn Thr Pro Ala Val Ile Asp Glu
    210                 215                 220

Ser Ala Asp Ile Lys Met Ala Val Asn Ser Ile Leu Leu Ser Lys Thr
225                 230                 235                 240

Phe Asp Asn Gly Met Ile Cys Ala Ser Glu Gln Ser Val Val Val Ala
                245                 250                 255

Ser Ser Ile Tyr Asp Glu Val Lys Lys Glu Phe Ala Asp Arg Gly Ala
            260                 265                 270

Tyr Ile Leu Ser Lys Asp Glu Thr Glu Lys Val Gly Lys Thr Ile Ile
        275                 280                 285

Ile Asn Gly Ala Leu Asn Ala Gly Ile Val Gly Gln Ser Ala Phe Lys
    290                 295                 300

Ile Ala Gln Met Ala Gly Val Ser Val Pro Glu Asp Ala Lys Val Leu
305                 310                 315                 320

Ile Gly Glu Val Lys Ser Val Glu Pro Glu Glu Glu Pro Phe Ala His
                325                 330                 335

Glu Lys Leu Ser Pro Val Leu Ala Met Tyr Lys Ala Lys Asp Phe Asp

340                 345                 350

Glu Ala Leu Leu Lys Ala Gly Arg Leu Val Glu Arg Gly Gly Ile Gly
        355                 360                 365

His Thr Ser Val Leu Tyr Val Asn Ala Met Thr Glu Lys Val Lys Val
    370                 375                 380

Glu Lys Phe Arg Glu Thr Met Lys Thr Gly Arg Thr Leu Ile Asn Met
385                 390                 395                 400

Pro Ser Ala Gln Gly Ala Ile Gly Asp Ile Tyr Asn Phe Lys Leu Ala
                405                 410                 415

Pro Ser Leu Thr Leu Gly Cys Gly Ser Trp Gly Gly Asn Ser Val Ser
            420                 425                 430

Glu Asn Val Gly Pro Lys His Leu Leu Asn Ile Lys Ser Val Ala Glu
        435                 440                 445

Arg Arg Glu Asn Met Leu Trp Phe Arg Val Pro Glu Lys Val Tyr Phe
    450                 455                 460

Lys Tyr Gly Ser Leu Gly Val Ala Leu Lys Glu Leu Arg Ile Met Glu
465                 470                 475                 480

Lys Lys Lys Ala Phe Ile Val Thr Asp Lys Val Leu Tyr Gln Leu Gly
                485                 490                 495

Tyr Val Asp Lys Ile Thr Lys Asn Leu Asp Glu Leu Arg Val Ser Tyr
            500                 505                 510

Lys Ile Phe Thr Asp Val Glu Pro Asp Pro Thr Leu Ala Thr Ala Lys
        515                 520                 525

Lys Gly Ala Ala Glu Leu Leu Ala Tyr Glu Pro Asp Thr Ile Ile Ala
    530                 535                 540

Val Gly Gly Gly Ser Ala Met Asp Ala Ala Lys Ile Met Trp Val Met
545                 550                 555                 560

Tyr Glu His Pro Glu Val Arg Phe Glu Asp Leu Ala Met Arg Phe Met
                565                 570                 575

Asp Ile Arg Lys Arg Val Tyr Val Phe Pro Lys Met Gly Glu Lys Ala
            580                 585                 590

Met Met Ile Ser Val Ala Thr Ser Ala Gly Thr Gly Ser Glu Val Thr
        595                 600                 605

Pro Phe Ala Val Ile Thr Asp Glu Arg Thr Gly Ala Lys Tyr Pro Leu
    610                 615                 620

Ala Asp Tyr Glu Leu Thr Pro Asn Met Ala Ile Val Asp Ala Glu Leu
625                 630                 635                 640

Met Met Gly Met Pro Lys Gly Leu Thr Ala Ala Ser Gly Ile Asp Ala
                645                 650                 655

Leu Thr His Ala Leu Glu Ala Tyr Val Ser Ile Met Ala Ser Glu Tyr
            660                 665                 670

Thr Asn Gly Leu Ala Leu Glu Ala Thr Arg Leu Val Phe Lys Tyr Leu
        675                 680                 685

Pro Ile Ala Tyr Thr Glu Gly Thr Thr Asn Val Lys Ala Arg Glu Lys
    690                 695                 700

Met Ala His Ala Ser Cys Ile Ala Gly Met Ala Phe Ala Asn Ala Phe
705                 710                 715                 720

Leu Gly Val Cys His Ser Met Ala His Lys Leu Gly Ala Gln His His
                725                 730                 735

Ile Pro His Gly Ile Ala Asn Ala Leu Met Ile Asp Glu Val Ile Lys
            740                 745                 750

Phe Asn Ala Val Glu Ala Pro Arg Lys Gln Ala Ala Phe Pro Gln Tyr
        755                 760                 765

Lys Tyr Pro Asn Val Lys Arg Arg Tyr Ala Arg Ile Ala Asp Tyr Leu
    770                 775                 780

Asn Leu Gly Gly Ser Thr Asp Asp Glu Lys Val Gln Phe Leu Ile Asn
785                 790                 795                 800

Ala Ile Asp Asp Leu Lys Thr Lys Leu Asn Ile Pro Lys Thr Ile Lys
                805                 810                 815

Glu Ala Gly Val Ser Glu Asp Lys Phe Tyr Ala Thr Leu Asp Thr Met
            820                 825                 830

Ser Glu Leu Ala Phe Asp Asp Gln Cys Thr Gly Ala Asn Pro Arg Tyr
        835                 840                 845

Pro Leu Ile Gly Glu Ile Lys Gln Met Tyr Ile Asn Ala Phe Asp Thr
    850                 855                 860

Pro Lys Ala Thr Val Glu Lys Lys Thr Arg Lys Lys Lys
865                 870                 875

<210>  8
<211>  2634
<212>  DNA
<213>  Clostridium ljungdahlii

<400>  8
atgaaggtaa ctaaggtaac taacgttgaa gaattaatga aaaagttaga tgaagtaacg      60

gctgctcaaa agaaattttc tagctatact caagaacaag tggatgaaat tttcaggcag     120

gcagctatgg cagccaatag tgctagaata gacttagcta aaatggcagt ggaagaaagc     180

ggaatgggaa ttgtagaaga caaggtcatt aaaaatcatt ttgttgcaga gtatatatat     240

aacaaatata agggtgaaaa aacctgtgga gttctggaac aagatgaagg ctttggtatg     300

gttagaattg cagaacctgt aggagttatt gcagcagtag tcccaacaac taatccaaca     360

tctacagcaa tatttaaatc actaatagct ttaaaaacta gaaatggtat agttttttcg      420

ccacatccaa gggcaaaaaa atcaactatt gcagcagcta agatagtact tgatgctgca     480

gttaaagctg gtgctcctga aggaattata ggatggatag atgaaccttc tattgaactt     540

tcacaggtgg taatgaaaga agcagatcta attcttgcaa ctggtggacc aggtatggtt     600

aaggctgcct attcttcagg aaagcctgct ataggagttg gtccaggtaa cacgcctgct     660

gtaattgatg aaagtgctga cattaaaatg gcagtaaatt caatactatt atcaaaaact     720

tttgataatg gtatgatttg tgcttcagag cagtcagtag tagttgcaag ctcaatatac     780

gatgaagtca agaaagagtt tgcagataga ggagcatata tattaagtaa ggatgaaaca     840

gagaaggttg gaaaaacaat tataattaat ggagccttaa atgctggcat tgtagggcaa     900

agtgctttta aaatagcaca gatggcagga gtgagtgtac cagaagatgc taaagtactt     960

ataggagaag ttaaatcagt agaaccggaa gaagagccct ttgcgcatga aaagctatct    1020

ccagttttag ctatgtacaa agcaaaagat tttgacgaag cactcctaaa ggctggaaga    1080

ttagttgaac gaggtggaat tgggcataca tctgtattat atgtaaatgc aatgacggaa    1140

aaagtaaagg tagaaaagtt cagagaaact atgaagactg gtagaacatt gataaatatg    1200

ccttcagcac aaggtgctat aggagatata tataacttta agctagctcc ttctttgaca    1260

ctaggttgtg gttcctgggg aggaaactct gtatcagaaa atgttggtcc taaacattta    1320

ttaaacataa agagtgttgc tgagaggaga gaaaatatgc tttggtttag agtacctgaa    1380

aaggtttatt tcaaatatgg tagtcttgga gttgcactaa aagaactgag aattatggag    1440

aagaaaaagg catttatagt aacggataaa gttctttatc aattaggtta tgtagataaa 1500

attacaaaaa atctggatga attaagagtt tcatataaaa tatttacaga tgtagaacca 1560

gatccaaccc ttgctacagc taaaaaaggt gcagcagaac tgttagctta tgaaccagat 1620

acaattatag cagtcggtgg tggttcagca atggatgcag ccaagatcat gtgggtaatg 1680

tatgagcatc cagaagtaag atttgaagat ttagctatga gatttatgga tataagaaag 1740

agagtgtatg ttttccctaa aatgggagaa aaggcaatga tgatttcagt agcaacatcc 1800

gcaggaacag ggtcggaagt tacgccattt gcagtaatta cggatgaaag aacaggagct 1860

aaatatcctc tggctgatta tgaattgact ccaaacatgg ctatagttga tgcagaactt 1920

atgatgggaa tgccaaaggg actaacagca gcttcaggta tagatgcatt aacccatgcg 1980

ctggaggcct atgtatcaat aatggcttca gaatatacca atggattggc tcttgaagca 2040

acaagattag tatttaaata tttgccaata gcttatacag aaggtacaac taatgtaaag 2100

gcaagagaaa aaatggctca tgcttcatgt attgcaggta tggcctttgc caatgcattt 2160

ttaggggtat gccactccat ggcacataaa ttgggagcac agcaccacat accacatgga 2220

attgccaatg cacttatgat agatgaagtt ataaagttca atgctgtaga ggctccaagg 2280

aaacaagcgg catttccaca atataaatat ccaaatgtta aaagaagata tgctagaata 2340

gctgattact taaatttagg tggaagtaca gatgatgaaa aagtacaatt tttaataaat 2400

gctatagatg acttgaaaac caagttaaat attccaaaga ctattaaaga agcgggagtt 2460

tcagaagata aattctatgc tactttagat acaatgtcag aactggcttt tgatgatcaa 2520

tgtacaggag ctaatccaag atatccatta ataggagaaa taaaacaaat gtatataaat 2580

gcatttgata caccaaaggc aactgtggag aagaaaacaa gaaagaaaaa ataa 2634

<210> 9
<211> 2634
<212> DNA
<213> artificial

<220>
<223> artificial sequence

<400> 9
atgaaagtga ccaaagtgac gaatgtggaa gaactgatga aaaaactgga tgaagttacc 60

gcagcccaga aaaaattcag cagctatacc caagaacagg tggatgaaat ttttcgtcag 120

gcagcaatgg cagcaaatag cgcacgtatt gatctggcaa aaatggcagt tgaagaaagc 180

ggtatgggta tcgttgaaga taaagtgatc aaaaaccact tcgtggccga gtacatctat 240

aacaaataca aaggtgaaaa aacctgcggt gttctggaac aggatgaagg ttttggtatg 300

gttcgtattg cagaaccggt tggtgttatt gcagcagttg ttccgaccac caatccgacc 360

agcaccgcaa tctttaaaag cctgattgca ctgaaaaccc gcaatggtat tgtttttagt 420

ccgcatccgc gtgcaaaaaa aagcaccatt gcagccgcaa aaattgttct ggatgcagcc 480

gttaaagcgg gtgcaccgga aggtattatt ggttggattg atgaaccgag cattgaactg 540

agccaggttg ttatgaaaga agccgatctg attctggcaa ccggtggtcc gggtatggtt 600

aaagcagcat atagcagcgg taaaccggca attggtgttg gtcctggtaa tacaccggca 660

gttattgatg aaagcgcaga cattaaaatg gccgtgaata gcattctgct gagcaaaacc 720

tttgataacg gtatgatttg tgcaagcgaa cagagcgttg ttgttgcaag cagcatttat 780

gacgaagtga aaaaagaatt tgcagatcgc ggtgcatata tcctgagcaa agatgaaacc 840

gaaaaagtgg gcaaaaccat cattattaac ggtgcactga atgccggtat tgttggtcag 900

agcgccttta aaatcgcaca gatggcaggc gttagcgttc cggaagatgc aaaagttctg 960

attggtgaag ttaaaagcgt tgaaccggaa gaggaaccgt ttgcacatga aaaactgagt 1020

ccggttctgg ccatgtataa agccaaagat tttgatgaag cactgctgaa agcaggtcgt 1080

ctggttgaac gtggtggtat tggtcatacc agcgttctgt atgttaatgc catgacggaa 1140

aaagtgaaag tcgaaaaatt ccgcgaaacc atgaaaaccg gtcgtaccct gattaatatg 1200

ccgagcgcac agggtgccat tggtgatatt tacaacttta aactggcacc gagcctgacc 1260

ctgggttgtg gtagctgggg tggtaatagc gttagcgaaa atgttggtcc gaaacatctg 1320

ctgaatatca aaagcgtggc agaacgtcgt gaaaatatgc tgtggtttcg tgttcctgag 1380

aaagtgtatt tcaaatatgg tagcctgggt gttgccctga aagaactgcg tattatggaa 1440

aaaaaaaaag ccttcatcgt gaccgacaaa gttctgtatc agctgggtta tgttgacaaa 1500

atcaccaaaa atctggacga actgcgtgtg agctataaaa tctttaccga tgttgaacct 1560

gatccgaccc tggccaccgc aaaaaaaggt gcagcagaac tgctggccta tgaaccggat 1620

accattattg cagttggtgg tggtagcgca atggatgctg ccaaaattat gtgggtgatg 1680

tatgaacatc cggaagtgcg ttttgaggac ctggcaatgc gttttatgga tattcgtaaa 1740

cgcgtgtatg tgtttccgaa aatgggtgaa aaagccatga tgattagcgt tgcaaccagc 1800

gcaggcaccg gtagcgaagt taccccgttt gcagttatta ccgatgaacg taccggtgca 1860

aaatatccgc tggcagatta tgaactgacc ccgaatatgg caattgttga tgcagagctg 1920

atgatgggta tgccgaaagg tctgaccgca gcaagcggta ttgatgcact gacccatgca 1980

ctggaagcct atgtgagcat tatggcaagc gaatatacaa atggtctggc cctggaagca 2040

acccgtctgg tgttcaaata tctgccgatt gcatataccg aaggcaccac caatgttaaa 2100

gcacgtgaaa aaatggccca tgcaagctgt attgcaggta tggcatttgc caatgcattt 2160

ctgggtgttt gtcatagcat ggcacataaa ctgggtgcac agcatcatat tccgcatggt 2220

atcgcaaatg cactgatgat cgatgaggtg attaaattca atgccgttga agcaccgcgt 2280

aaacaggcag catttccgca gtataaatac ccgaatgtta aacgtcgtta tgcccgtatt 2340

gccgattatc tgaatctggg tggtagcacc gatgatgaga aagttcagtt tctgattaac 2400

gccatcgatg atctgaaaac caaactgaat attccgaaaa ccattaaaga agcaggggtc 2460

agcgaagata aattctatgc aaccctggat accatgagcg aactggcatt tgatgatcag 2520

tgtacaggtg caaatccgcg ttatccgctg attggcgaaa tcaaacaaat gtatatcaac 2580

gcctttgata ccccgaaagc aaccgttgag aaaaaaaccc gtaaaaaaaa ataa 2634

<210> 10
<211> 375
<212> PRT
<213> Equus caballus

<400> 10

Met Ser Thr Ala Gly Lys Val Ile Lys Cys Lys Ala Ala Val Leu Trp
1               5                   10                  15

Glu Glu Lys Lys Pro Phe Ser Ile Glu Glu Val Glu Val Ala Pro Pro
            20                  25                  30

Lys Ala His Glu Val Arg Ile Lys Met Val Ala Thr Gly Ile Cys Arg
        35                  40                  45

Ser Asp Asp His Val Val Ser Gly Thr Leu Val Thr Pro Leu Pro Val
    50                  55                  60

Ile Ala Gly His Glu Ala Ala Gly Ile Val Glu Ser Ile Gly Glu Gly
65                  70                  75                  80

Val Thr Thr Val Arg Pro Gly Asp Lys Val Ile Pro Leu Phe Thr Pro
                85                  90                  95

Gln Cys Gly Lys Cys Arg Val Cys Lys His Pro Glu Gly Asn Phe Cys
            100                 105                 110

Leu Lys Asn Asp Leu Ser Met Pro Arg Gly Thr Met Gln Asp Gly Thr
        115                 120                 125

Ser Arg Phe Thr Cys Arg Gly Lys Pro Ile His His Phe Leu Gly Thr
    130                 135                 140

Ser Thr Phe Ser Gln Tyr Thr Val Val Asp Glu Ile Ser Val Ala Lys
145                 150                 155                 160

Ile Asp Ala Ala Ser Pro Leu Glu Lys Val Cys Leu Ile Gly Cys Gly
                165                 170                 175

Phe Ser Thr Gly Tyr Gly Ser Ala Val Lys Val Ala Lys Val Thr Gln
            180                 185                 190

Gly Ser Thr Cys Ala Val Phe Gly Leu Gly Gly Val Gly Leu Ser Val
        195                 200                 205

Ile Met Gly Cys Lys Ala Ala Gly Ala Ala Arg Ile Ile Gly Val Asp
    210                 215                 220

Ile Asn Lys Asp Lys Phe Ala Lys Ala Lys Glu Val Gly Ala Thr Glu
225                 230                 235                 240

Cys Val Asn Pro Gln Asp Tyr Lys Lys Pro Ile Gln Glu Val Leu Thr
                245                 250                 255

Glu Met Ser Asn Gly Gly Val Asp Phe Ser Phe Glu Val Ile Gly Arg
            260                 265                 270

Leu Asp Thr Met Val Thr Ala Leu Ser Cys Cys Gln Glu Ala Tyr Gly
        275                 280                 285

Val Ser Val Ile Val Gly Val Pro Pro Asp Ser Gln Asn Leu Ser Met
    290                 295                 300

Asn Pro Met Leu Leu Leu Ser Gly Arg Thr Trp Lys Gly Ala Ile Phe
305                 310                 315                 320

Gly Gly Phe Lys Ser Lys Asp Ser Val Pro Lys Leu Val Ala Asp Phe
                325                 330                 335

Met Ala Lys Lys Phe Ala Leu Asp Pro Leu Ile Thr His Val Leu Pro
            340                 345                 350

Phe Glu Lys Ile Asn Glu Gly Phe Asp Leu Leu Arg Ser Gly Glu Ser
        355                 360                 365

Ile Arg Thr Ile Leu Thr Phe
    370                 375

<210>  11
<211>  1128
<212>  DNA
<213>  Equus caballus

<400>  11
atgagcacag caggaaaagt aataaaatgc aaagcggctg tgctgtggga ggaaaagaaa      60

ccattttcca tcgaggaggt ggaggttgca cccccgaagg cccatgaagt ccgtataaag     120

atggtggcca caggaatttg tcgctcagat gaccacgtgg ttagtggaac ccttgtcaca 180

cctcttcctg tgatcgcagg ccatgaggca gcgggcattg tggagagcat tggagaaggc 240

gtcactacag taagaccagg tgataaagtc atcccactct ttactcccca gtgtggaaaa 300

tgcagggttt gtaagcaccc tgaaggcaac ttctgcttga aaaatgatct gagcatgcct 360

cggggaacca tgcaggatgg taccagcagg ttcacctgca gagggaagcc catccaccac 420

ttccttggca ccagcacctt ctcccagtac accgtggtgg acgagatctc agtggccaag 480

atcgatgcgg cctcaccgct ggagaaagtc tgtctcattg gctgtggatt ttctactggt 540

tatgggtctg cagtcaaggt tgccaaggtc acccagggct ccacctgtgc cgtgtttggc 600

cttggaggag tgggcctgtc tgttatcatg ggctgtaaag cagccggagc ggccaggatc 660

attggggtgg acatcaacaa agacaagttt gcaaaggcca aagaagtggg tgccactgag 720

tgtgtcaacc ctcaggacta caagaaaccc atccaggagg tgctgacaga aatgagcaat 780

ggaggtgtgg atttttcctt tgaagtcatt ggtcggctcg acactatggt gactgccttg 840

tcatgctgtc aagaagcata tggtgtgagc gtcattgtgg gagtacctcc tgattcccaa 900

aatctctcta tgaatcctat gttgctactg agtggacgta cctggaaagg agctattttt 960

ggcggtttta agagtaaaga ttctgtcccc aaacttgtgg ccgattttat ggctaaaaag 1020

tttgcactgg atcctttaat cacccatgtt ttaccttttg aaaaaataaa tgaaggattt 1080

gacctgcttc gctctggaga gagtatccgt accatcctga cgttttga 1128

<210> 12
<211> 1128
<212> DNA
<213> artificial

<220>
<223> artificial sequence

<400> 12
atgtctaccg ctggtaaagt tatcaaatgc aaagctgctg ttctgtggga agaaaaaaaa 60

ccgttctcta tcgaagaagt tgaagttgct ccgccgaaag ctcacgaagt tcgtatcaaa 120

atggttgcta ccggtatctg ccgttctgac gaccacgttg tttctggtac cctggttacc 180

ccgctgccgg ttatcgctgg tcacgaagct gctggtatcg ttgaatctat cggtgaaggt 240

gttaccaccg ttcgtccggg tgacaaagtt atcccgctgt tcaccccgca gtgcggtaaa 300

tgccgtgttt gcaaacaccc ggaaggtaac ttctgcctga aaaacgacct gtctatgccg 360

cgtggtacca tgcaggacgg tacctctcgt ttcacctgcc gtggtaaacc gatccaccac 420

ttcctgggta cctctacctt ctctcagtac accgttgttg acgaaatctc tgttgctaaa 480

atcgacgctg cttctccgct ggaaaaagtt tgcctgatcg gttgcggttt ctctaccggt 540

tacggttctg ctgttaaagt tgctaaagtt acccagggtt ctacctgcgc tgttttcggt 600

ctgggtggtg ttggtctgtc tgttatcatg ggttgcaaag ctgctggtgc tgctcgtatc 660

atcggtgttg acatcaacaa agacaaattc gctaaagcta aagaagttgg tgctaccgaa 720

tgcgttaacc cgcaggacta caaaaaaccg atccaggaag ttctgaccga aatgtctaac 780

ggtggtgttg acttctcttt cgaagttatc ggtcgtctgg acaccatggt taccgctctg 840

tcttgctgcc aggaagctta cggtgtttct gttatcgttg gtgttccgcc ggactctcag 900

aacctgtcta tgaacccgat gctgctgctg tctggtcgta cctggaaagg tgctatcttc 960

ggtggtttca aatctaaaga ctctgttccg aaactggttg ctgacttcat ggctaaaaaa 1020

ttcgctctgg acccgctgat cacccacgtt ctgccgttcg aaaaaatcaa cgaaggtttc 1080

gacctgctgc gttctggtga atctatccgt accatcctga ccttctaa 1128

<210> 13
<211> 352
<212> PRT
<213> Thermoanaerobacter brockii

<400> 13

Met Lys Gly Phe Ala Met Leu Ser Ile Gly Lys Val Gly Trp Ile Glu
1               5                   10                  15

Lys Glu Lys Pro Ala Pro Gly Pro Phe Asp Ala Ile Val Arg Pro Leu
            20                  25                  30

Ala Val Ala Pro Cys Thr Ser Asp Ile His Thr Val Phe Glu Gly Ala
        35                  40                  45

Ile Gly Glu Arg His Asn Met Ile Leu Gly His Glu Ala Val Gly Glu
    50                  55                  60

Val Val Glu Val Gly Ser Glu Val Lys Asp Phe Lys Pro Gly Asp Arg
65                  70                  75                  80

Val Val Val Pro Ala Ile Thr Pro Asp Trp Arg Thr Ser Glu Val Gln
                85                  90                  95

Arg Gly Tyr His Gln His Ser Gly Gly Met Leu Ala Gly Trp Lys Phe
            100                 105                 110

Ser Asn Val Lys Asp Gly Val Phe Gly Glu Phe Phe His Val Asn Asp
        115                 120                 125

Ala Asp Met Asn Leu Ala His Leu Pro Lys Glu Ile Pro Leu Glu Ala
    130                 135                 140

Ala Val Met Ile Pro Asp Met Met Thr Thr Gly Phe His Gly Ala Glu
145                 150                 155                 160

Leu Ala Asp Ile Glu Leu Gly Ala Thr Val Ala Val Leu Gly Ile Gly
                165                 170                 175

Pro Val Gly Leu Met Ala Val Ala Gly Ala Lys Leu Arg Gly Ala Gly
            180                 185                 190

Arg Ile Ile Ala Val Gly Ser Arg Pro Val Cys Val Asp Ala Ala Lys
        195                 200                 205

Tyr Tyr Gly Ala Thr Asp Ile Val Asn Tyr Lys Asp Gly Pro Ile Glu
    210                 215                 220

Ser Gln Ile Met Asn Leu Thr Glu Gly Lys Gly Val Asp Ala Ala Ile
225                 230                 235                 240

Ile Ala Gly Gly Asn Ala Asp Ile Met Ala Thr Ala Val Lys Ile Val
                245                 250                 255

Lys Pro Gly Gly Thr Ile Ala Asn Val Asn Tyr Phe Gly Glu Gly Glu
            260                 265                 270

Val Leu Pro Val Pro Arg Leu Glu Trp Gly Cys Gly Met Ala His Lys
        275                 280                 285

Thr Ile Lys Gly Gly Leu Cys Pro Gly Gly Arg Leu Arg Met Glu Arg
    290                 295                 300

Leu Ile Asp Leu Val Phe Tyr Lys Arg Val Asp Pro Ser Lys Leu Val
305                 310                 315                 320

Thr His Val Phe Arg Gly Phe Asp Asn Ile Glu Lys Ala Phe Met Leu
                325                 330                 335

Met Lys Asp Lys Pro Lys Asp Leu Ile Lys Pro Val Val Ile Leu Ala
            340                 345                 350

<210>  14
<211>  1059
<212>  DNA
<213>  Thermoanaerobacter brockii

<400>  14
atgaaaggtt ttgcaatgct cagtatcggt aaagttggct ggattgagaa ggaaaagcct      60

gctcctggcc catttgatgc tattgtaaga cctctagctg tggccccttg cacttcggac     120

attcataccg tttttgaagg cgccattggc gaaagacata acatgatact cggtcacgaa     180

gctgtaggtg aagtagttga agtaggtagt gaggtaaaag attttaaacc tggtgatcgc 240

gttgttgtgc cagctattac ccctgattgg cggacctctg aagtacaaag aggatatcac 300

cagcactccg gtggaatgct ggcaggctgg aaattttcga atgtaaaaga tggtgttttt 360

ggtgaatttt ttcatgtgaa tgatgctgat atgaatttag cacatctgcc taaagaaatt 420

ccattggaag ctgcagttat gattcccgat atgatgacca ctggttttca cggagctgaa 480

ctggcagata tagaattagg tgcgacggta gcagttttgg gtattggccc agtaggtctt 540

atggcagtcg ctggtgccaa attgcgtgga gccggaagaa ttattgccgt aggcagtaga 600

ccagtttgtg tagatgctgc aaaatactat ggagctactg atattgtaaa ctataaagat 660

ggtcctatcg aaagtcagat tatgaatcta actgaaggca aaggtgtcga tgctgccatc 720

atcgctggag gaaatgctga cattatggct acagcagtta agattgttaa acctggtggc 780

accatcgcta atgtaaatta ttttggcgaa ggagaggttt tgcctgttcc tcgtcttgaa 840

tggggttgcg gcatggctca taaaactata aaaggcgggc tatgccccgg tggacgtcta 900

agaatggaaa gactgattga ccttgttttt tataagcgtg tcgatccttc taagctcgtc 960

actcacgttt tccggggatt tgacaatatt gaaaaagcct ttatgttgat gaaagacaaa 1020

ccaaaagacc taatcaaacc tgttgtaata ttagcataa 1059

<210> 15
<211> 490
<212> PRT
<213> Clostridium aminobutyricum

<400> 15

Met Leu Met Thr Ala Glu Gln Tyr Ile Glu Ser Leu Arg Lys Leu Asn
1               5                   10                  15

Thr Arg Val Tyr Met Phe Gly Glu Lys Ile Glu Asn Trp Val Asp His
            20                  25                  30

Pro Met Ile Arg Pro Ser Ile Asn Cys Val Ala Met Thr Tyr Glu Leu
        35                  40                  45

Ala Gln Asp Pro Gln Tyr Ala Asp Leu Met Thr Thr Lys Ser Asn Leu
    50                  55                  60

Ile Gly Lys Thr Ile Asn Arg Phe Ala Asn Leu His Gln Ser Thr Asp
65                  70                  75                  80

Asp Leu Arg Lys Lys Val Lys Met Gln Arg Leu Leu Gly Gln Lys Thr
                85                  90                  95

Ala Ser Cys Phe Gln Arg Cys Val Gly Met Asp Ala Phe Asn Ala Val
            100                 105                 110
Phe Ser Thr Thr Tyr Glu Ile Asp Gln Lys Tyr Gly Thr Asn Tyr His
        115                 120                 125
Lys Asn Phe Thr Glu Tyr Leu Lys Tyr Ile Gln Glu Asn Asp Leu Ile
    130                 135                 140
Val Asp Gly Ala Met Thr Asp Pro Lys Gly Asp Arg Gly Leu Ala Pro
145                 150                 155                 160
Ser Ala Gln Lys Asp Pro Asp Leu Phe Leu Arg Ile Val Glu Lys Arg
                165                 170                 175
Glu Asp Gly Ile Val Val Arg Gly Ala Lys Ala His Gln Thr Gly Ser
            180                 185                 190
Ile Asn Ser His Glu His Ile Ile Met Pro Thr Ile Ala Met Thr Glu
        195                 200                 205
Ala Asp Lys Asp Tyr Ala Val Ser Phe Ala Cys Pro Ser Asp Ala Asp
    210                 215                 220
Gly Leu Phe Met Ile Tyr Gly Arg Gln Ser Cys Asp Thr Arg Lys Met
225                 230                 235                 240
Glu Glu Gly Ala Asp Ile Asp Leu Gly Asn Lys Gln Phe Gly Gly Gln
                245                 250                 255
Glu Ala Leu Val Val Phe Asp Asn Val Phe Ile Pro Asn Asp Arg Ile
            260                 265                 270
Phe Leu Cys Gln Glu Tyr Asp Phe Ala Gly Met Met Val Glu Arg Phe
        275                 280                 285
Ala Gly Tyr His Arg Gln Ser Tyr Gly Gly Cys Lys Val Gly Val Gly
    290                 295                 300
Asp Val Val Ile Gly Ala Ala Ala Leu Ala Ala Asp Tyr Asn Gly Ala
305                 310                 315                 320
Gln Lys Ala Ser His Val Lys Asp Lys Leu Ile Glu Met Thr His Leu
                325                 330                 335
Asn Glu Thr Leu Tyr Cys Cys Gly Ile Ala Cys Ser Ala Glu Gly Tyr
            340                 345                 350

Pro Thr Ala Ala Gly Asn Tyr Gln Ile Asp Leu Leu Leu Ala Asn Val
        355                 360                 365

Cys Lys Gln Asn Ile Thr Arg Phe Pro Tyr Glu Ile Val Arg Leu Ala
    370                 375                 380

Glu Asp Ile Ala Gly Gly Leu Met Val Thr Met Pro Ser Glu Ala Asp
385                 390                 395                 400

Phe Lys Ser Glu Thr Val Val Gly Arg Asp Gly Glu Thr Ile Gly Asp
                405                 410                 415

Phe Cys Asn Lys Phe Phe Ala Ala Ala Pro Thr Cys Thr Thr Glu Glu
            420                 425                 430

Arg Met Arg Val Leu Arg Phe Leu Glu Asn Ile Cys Leu Gly Ala Ser
        435                 440                 445

Ala Val Gly Tyr Arg Thr Glu Ser Met His Gly Ala Gly Ser Pro Gln
    450                 455                 460

Ala Gln Arg Ile Met Ile Ala Arg Gln Gly Asn Ile Asn Ala Lys Lys
465                 470                 475                 480

Glu Leu Ala Lys Ala Ile Ala Gly Ile Lys
                485                 490

<210>  16
<211>  1473
<212>  DNA
<213>  Clostridium aminobutyricum

<400>  16
atgttaatga cagcagaaca gtacattgag agtctaagaa agctaaacac aagagtttat      60

atgtttggtg aaaaaatcga gaattgggtg gatcatccaa tgatcagacc ttccatcaac     120

tgcgtagcaa tgacttatga attagctcag gatcctcagt acgctgactt aatgactaca     180

aagtcaaact taataggtaa aactatcaac agatttgcaa atctacacca gagcacagat     240

gaccttagaa aaaaggttaa gatgcagaga cttcttggac agaagaccgc atcatgcttc     300

cagagatgtg taggtatgga cgctttcaat gcagttttct caactacata tgaaatcgac     360

cagaaatatg gaacaaacta tcacaagaac tttactgaat acttaaagta tatacaggaa     420

aatgacctta ttgttgacgg tgcaatgact gaccctaagg gtgacagagg acttgctcca     480

tccgcacaga aggatccaga tcttttcttg agaatcgttg aaaaaagaga agatggtatc     540

gttgtaagag gagctaaggc tcaccagact ggttccatca actcccacga acacatcatc     600

atgcctacaa tcgctatgac agaagctgat aaggattatg cagtatcatt tgcttgtcct 660

tccgatgctg atggtctatt catgatctac ggcagacagt catgtgacac aagaaagatg 720

gaagaaggcg ctgacattga ccttggtaac aagcagttcg gcggacagga agctttagtc 780

gtattcgata acgtatttat tccaaatgac agaatcttcc tttgccaaga atatgatttc 840

gctggcatga tggtagaaag atttgctgga taccacagac agtcatacgg cggatgtaag 900

gttggagtag gcgacgttgt aatcggtgct gctgctttag ctgctgacta caatggagct 960

cagaaggctt ctcacgttaa agataagctt atcgaaatga ctcacttaaa tgaaacttta 1020

tattgctgcg gtattgcttg ttcagcagaa ggttatccaa ctgctgctgg taactatcag 1080

attgaccttc ttcttgcaaa tgtatgtaag cagaacatca ctagattccc ttacgaaatc 1140

gtaagactag ctgaagatat cgctggtgga ttaatggtta ctatgccttc agaagctgac 1200

tttaagtcag aaacagttgt tggtagagat ggcgaaacta ttggagattt ctgcaataag 1260

ttcttcgctg ctgctcctac ttgcacaaca gaagaaagaa tgagagttct tagattctta 1320

gaaaacatct gcttaggtgc atccgctgta ggttacagaa ctgaatccat gcatggtgca 1380

ggttcccctc aggctcagag aatcatgatc gctcgtcagg gcaacatcaa cgctaagaaa 1440

gaattagcta aggcaatcgc tggaattaaa taa 1473

<210> 17
<211> 1473
<212> DNA
<213> artificial

<220>
<223> artificial sequence

<400> 17
atgctgatga ccgcagaaca gtatattgaa agcctgcgta aactgaatac ccgtgtgtat 60

atgtttggcg agaaaattga aaactgggtg gatcatccga tgattcgtcc gagcattaat 120

tgtgttgcaa tgacctatga actggcacag gacccgcagt atgccgatct gatgaccacc 180

aaaagcaatc tgattggcaa aaccattaac cgctttgcaa atctgcatca gagcaccgat 240

gatctgcgta aaaaagttaa aatgcagcgt ctgctgggtc agaaaaccgc aagctgtttt 300

cagcgttgtg ttggtatgga tgcatttaat gcagttttca gcaccaccta tgagatcgat 360

cagaaatatg gcaccaacta ccacaaaaac tttaccgagt atctgaaata catccaagag 420

aacgatctga ttgttgatgg tgccatgacc gatccgaaag gtgatcgtgg tctggcaccg 480

agcgcacaga aagatccgga cctgtttctg cgtattgttg aaaaacgtga agatggtatt 540

gttgtgcgtg gtgcaaaagc acatcagacc ggtagcatta atagccatga acatattatc 600

atgccgacca ttgctatgac cgaagccgat aaagattatg cagttagctt tgcatgtccg 660

tcagatgcag atggtctgtt tatgatttat ggtcgtcaga gctgtgatac ccgtaaaatg 720

gaagaaggtg cagatattga tctgggcaat aaacagtttg gtggccaaga agcactggtc 780

gtttttgata atgtgtttat tccgaacgat cgcatttttc tgtgccaaga atatgatttt 840

gccggtatga tggttgaacg ttttgcaggt tatcatcgtc agagttatgg tggttgtaaa 900

gttggtgttg gtgatgttgt tattggtgca gcagcactgg cagcagatta taatggtgcc 960

cagaaagcaa gccatgtgaa agataaactg atcgaaatga cccatctgaa cgaaaccctg 1020

tattgttgcg gtattgcatg tagcgcagaa ggttatccga ccgcagcagg taattatcag 1080

attgatctgc tgctggccaa tgtgtgcaaa cagaatatta cccgttttcc gtatgaaatt 1140

gttcgtctgg ccgaagatat tgccggtggt ctgatggtta ccatgccgag cgaagcagat 1200

ttcaaaagcg aaaccgttgt tggtcgtgat ggtgaaacca ttggtgattt ttgcaacaaa 1260

tttttcgcag ccgcaccgac ctgtaccacc gaagaacgta tgcgtgttct gcgttttctg 1320

gaaaacattt gtctgggtgc aagcgcagtt ggttatcgta ccgaaagcat gcatggtgca 1380

ggtagtccgc aggcacagcg tattatgatt gcacgtcagg gtaacattaa cgccaaaaaa 1440

gaactggcca aagcaattgc gggtatcaaa taa 1473

<210> 18
<211> 269
<212> PRT
<213> Streptomyces collinus

<400> 18

Met Arg His Ala Asp Thr Val Leu Tyr Glu Val Ser Asp Gly Leu Ala
1               5                   10                  15

Thr Ile Thr Leu Asn Arg Pro Glu Ala Met Asn Ala Leu Asn Ile Ala
            20                  25                  30

Thr Lys Val Ala Leu Arg Glu Ala Ala Glu Ser Ala Ala Ala Asp Thr
        35                  40                  45

Ala Val Arg Ala Ile Leu Leu Thr Ala Ala Gly Glu Arg Ala Phe Cys
    50                  55                  60

Val Gly Gln Asp Leu Lys Glu His Ile Gly Leu Leu Ala Gln Asp Arg
65                  70                  75                  80

Glu Thr Gly Ser Gly Gln Thr Met Ser Thr Val Lys Glu His Tyr Asn
                85                  90                  95

Pro Ile Val Arg Ala Leu Ala Gly Ala Ala Lys Pro Val Val Ala Ala
            100                 105                 110

Val Asn Gly Val Ala Ala Gly Ala Gly Phe Gly Phe Ala Leu Thr Ala
        115                 120                 125

Asp Tyr Arg Val Val Ala Asp Thr Ala Ser Phe Asn Thr Ser Phe Ala
    130                 135                 140

Gly Val Ala Leu Thr Ala Asp Ser Gly Ile Ser Trp Thr Leu Pro Arg
145                 150                 155                 160

Val Ile Gly Pro Ser Arg Ala Ala Asp Leu Leu Leu Phe Pro Arg Ser
                165                 170                 175

Ile Ser Ala Gln Asp Ala Leu Glu Leu Gly Ile Ala Asn Arg Val Val
            180                 185                 190

Pro Ala Ala Glu Leu Arg Ala Glu Ala Glu Lys Val Ala Arg Ala Leu
        195                 200                 205

Ala Glu Gly Pro Thr Val Ala Tyr Ala Ala Ile Lys Glu Ala Val Ala
    210                 215                 220

Tyr Gly Leu Thr His Ser Leu Ala Glu Thr Leu Glu Lys Glu Asp Glu
225                 230                 235                 240

Leu Gln Thr Arg Ala Gly Gln Ser Glu Asp His Ala Ile Val Val Gln
                245                 250                 255

Ala Phe Val Asn Lys Glu Arg Pro Lys Tyr Leu Gly Arg
            260                 265

<210>  19
<211>  810
<212>  DNA
<213>  Streptomyces collinus

<400>  19
atgcgccatg ccgacaccgt gctctacgag gtgagcgacg ggctcgcgac gatcacgctg      60

aaccgccccg aggcgatgaa cgcgctgaac atcgcgacca aggtcgccct ccgcgaggcg     120

gcggaatcgg cggccgccga cacggccgta cgggcgattc tgctgaccgc ggccggggag     180

cgggcgttct gcgtgggcca ggacctcaag gagcacatcg ggctgctcgc ccaggaccgg     240

gagaccgggt cggggcagac gatgagcacg gtcaaggagc actacaaccc gatcgtgcgg     300

gccctcgccg gggcggcgaa gccggtggtc gccgcggtga acggcgtcgc ggccggggcc     360

ggcttcggct tcgcgctcac cgcggactac cgcgtcgtcg ccgacaccgc ctccttcaac     420

acgtccttcg ccggcgtggc cctcaccgcc gactccggca tctcctggac gctgccccgt     480

gtgatcggcc cgagccgcgc cgccgacctg ctgctcttcc cgcgcagcat cagcgcccag     540

gacgcgctgg agctgggcat cgccaaccgg gtcgtccccg ccgcggagct gcgcgccgag 600

gccgagaagg tggcgcgggc gctggccgag gggccgacgg tggcgtacgc ggcgatcaag 660

gaggcggtgg cctacgggct gacgcactcg ctcgcggaga cgctggagaa ggaggacgag 720

ctgcagaccc gggccgggca gtccgaggac cacgccatcg tcgtccaggc gttcgtgaac 780

aaggagcggc ccaagtacct ggggcggtag 810

<210> 20
<211> 810
<212> DNA
<213> artificial

<220>
<223> artificial sequence

<400> 20
atgcgtcatg cagataccgt tctgtatgaa gttagtgatg gtctggcaac cattaccctg 60

aatcgtccgg aagcaatgaa tgcactgaat attgcaacca aagttgcact gcgtgaagca 120

gcagaaagcg cagcagccga taccgcagtt cgtgcaattc tgctgaccgc agccggtgaa 180

cgtgcatttt gtgttggtca ggatctgaaa gaacatattg gtctgctggc acaggatcgt 240

gaaaccggta gcggtcagac catgagcacc gttaaagaac attataatcc gattgttcgt 300

gcactggcag gcgcagcaaa accggttgtg gcagcagtta atggtgttgc agcgggtgca 360

ggttttggtt ttgcactgac agcagattat cgtgttgttg cggataccgc cagctttaat 420

accagctttg ccggtgttgc cctgacagcc gatagcggta ttagctggac cctgcctcgt 480

gttattggtc cgagccgtgc agccgatctg ctgctgtttc cgcgtagcat tagcgcacag 540

gatgcactgg aactgggtat tgcaaatcgt gtggttccgg cagcagaact gcgtgcagaa 600

gccgaaaaag ttgcccgtgc cctggcagaa ggtccgaccg ttgcctatgc agcaattaaa 660

gaagcagttg catacggtct gacccatagc ctggccgaaa ccctggaaaa agaagatgaa 720

ctgcaaaccc gtgcaggtca gagcgaagat catgcaattg ttgttcaggc ctttgtgaat 780

aaagagcgtc cgaaatatct gggtcgttaa 810

## Beschreibung

Die vorliegende Erfindung betrifft ein Verfahren zur Herstellung von 3-Butenal und vorzugsweise von Vinylethanol (3-Buten-1-ol) mit Hilfe von Enzymen.

### Hintergrund der Erfindung

3-Butenal und insbesondere Vinylethanol (3-Buten-1-ol) sind Vorstufen von 1,3-Butadien. 1,3-Butadien, oder einfach Butadien genannt, ist ein wichtiges Monomer für synthetische Kautschuke wie Styrol-Butadien-Kautschuk (SBR), Acrylontril-Butadien (NBR), Kunststoffe, einschließlich Polybutadien (PB), Acrylnitril-Butadien-Styrol (ABS) und als Ausgangsmaterial für Adipodinitril für Nylon-6,6 und andere Substanzen. Butadien wird technisch in erheblichen Mengen durch Wasserstoffabspaltung aus gesättigten Kohlenwasserstoffen bei Dampfkrackverfahren hergestellt und aus den Cracker-Strömen durch Extraktion isoliert.

Butadien kann auch unter Anwendung von anderen Methoden wie durch Dehydrierung von n-Butan hergestellt werden oder durch Dehydrierung von n-Butanol oder Butandiol.

Heute werden die Monomere ausschließlich aus fossilen Rohstoffen hergestellt. Die industrielle Produktion von Butadien unter Nutzung der oben genannten Verfahren ist jedoch nicht nur von der kontinuierlichen Zufuhr fossiler Ausgangstoffe abhängig, sondern beinhaltet auch den Einsatz von ggf. aggressiven und unweltschädlichen Chemikalien. Um die Abhängigkeit von fossilen Rohstoffen als Edukte für die industrielle Synthesen zu verringern, werden gegenwärtig zahlreiche Ansätze entwickelt, die darauf abzielen, in der Industrie verwendete Chemikalien auf der Basis nachwachsender Rohstoffe zu erzeugen. Daher sollen neue biotechnologische Verfahren zur Herstellung von 3-Butenal, Vinylethanol (3-Buten-1-ol) und schließlich von Butadien bereitgestellt werden, die umweltfreundlicher und weniger Energie-, Wasser- und Abfall-intensiv sind.

Die Aufgabe der vorliegenden Erfindung war es daher, biotechnologische Verfahren für die Herstellung bekannter Vorstufen für die Synthese von Kautschuken durch stoffliche Nutzung nachwachsender einheimischer Rohstoffe bereitzustellen. Als Ausgangsstoffe sollen nachwachsende Rohstoffe direkt als Kohlenhydrate oder in Form von Synthesegas aus einer Biomassevergasung genutzt und unter Einsatz von maßgeschneiderten carboxydotrophen Mikroorganismen umgesetzt werden können. Insbesondere war die der vorliegenden Anmeldung zugrunde liegende Aufgabe, neue Verfahren zu finden, mit denen mit Hilfe von Enzymen Vorstufen von 1,3-Butadien, nämlich Vinylacetaldehyd (3-Butenal) und Vinylethanol (3-Buten-1-ol) bereitgestellt werden können.

### Zusammenfassung der Erfindung

Die technische Aufgabe wurde gelöst durch ein Verfahren zur Umsetzung einer Verbindung mit der Formel (II) [Vinylacetyl-CoA, 3-Butenoyl-CoA] 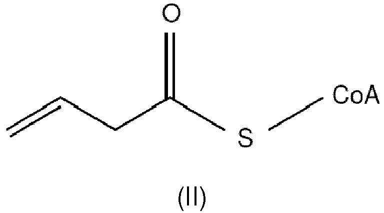 durch Kontaktieren der Verbindung mit der Formel (II) mit einer Aldehyd-Dehydrogenase-Aktivität, wobei eine Verbindung mit der Formel (III) [Vinylacetaldehyd, 3-Butenal] erhalten wird

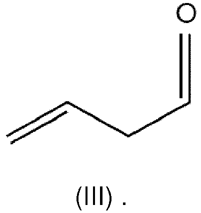

Dabei ist bevorzugt, dass die Aldehyd-Dehydrogenase-Aktivität eine acylierende Aldehyd-Dehydrogenase-Aktivität ist. In einem weiteren bevorzugten Verfahren geht die Aldehyd-Dehydrogenase-Aktivität von einer acylierenden Acetaldehyd-Dehydrogenase aus.

Grundsätzlich sind für die Umsetzung Vinylacetyl-CoA zu Vinylacetaldehyd alle Aldehyd-Dehydrogenasen geeignet, die in der Lage sind, die Reduktion von Acyl-CoA-Verbindungen zu Aldehyden zu katalysieren. Diese Enzyme werden auch als acylierende Aldehyd-Dehydrogenasen bezeichnet oder als Acetaldehyd:NAD⁺ Oxidoreduktasen (CoA acetylierend) charakterisiert. Insbesondere sind acylierende Aldehyd-Dehydrogenasen für die Umsetzung geeignet, die gemäß IUBMB Nomenklatur mit EC 1.2.1.10 bezeichnet werden.

Die Aldehyd-Dehydrogenase-Aktivität geht von einer Aldehyd-Dehydrogenase aus, die insbesondere ausgewählt ist aus der Gruppe bestehend aus *Pseudomonas sp.* DmpF (SEQ ID NO: 1), *Comamonas testosteroni* AcDH (SEQ ID NO: 4) und *Clostridium Ijungdahlii* AdhE2 (SEQ ID NO: 7) oder Varianten davon. Dabei ist das letztere Enzym bifunktional und weist noch eine Alkohol-Dehydrogenase-Aktivität auf. Die Aldehyd-Dehydrogenasen *Pseudomonas sp.* DmpF (SEQ ID NO: 1), *Comamonas testosteroni AcDH* (SEQ ID NO: 4) und *Clostridium ljungdahlii* AdhE2 (SEQ ID NO: 7) sind acylierende Aldehyd-Dehydrogenasen bzw. Acetaldehyd:NAD⁺ Oxidoreduktasen (CoA acetylierend) und werden gemäß der IUBMB Nomenklatur entsprechend mit der Nummer EC 1.2.1.10 klassifiziert.

Insbesondere ist das Enzym CT-AcDH geeignet und bevorzugt, da es eine Selektivität gegenüber Vinylacetyl-CoA im Vergleich zu Crotonyl-CoA aufweist.

In einem weiteren bevorzugten Verfahren weist die Acetaldehyd-Dehydrogenase eine der in irgendeiner der SEQ ID NOs: 1, 4 und 7 gezeigten Aminosäuresequenzen auf oder eine Aminosäuresequenz, deren Aminosäurepositionen mindestens zu 15 %, bevorzugt mindestens zu 25 %, weiter bevorzugt zu mindestens 50 % und noch weiter bevorzugt zu mindestens 60 % identisch sind mit irgendeiner der in SEQ ID NOs: 1, 4 und 7 gezeigten Aminosäuresequenzen.

In einem bevorzugten Verfahren wird die Verbindung mit der Formel (III) weiter umgesetzt zu einer Verbindung mit der Formel (IV) [Vinylethanol, 3-Buten-1-ol] 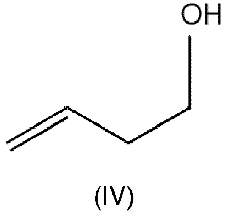 durch Kontaktieren der Verbindung mit der Formel (III) mit einer Alkohol-Dehydrogenase-Aktivität.

Die Alkohol-Dehydrogenase-Aktivität geht insbesondere von einer Alkohol-Dehydrogenase aus. Grundsätzlich sind für die Umsetzung von Vinylacetaldehyd zu Vinylalkohol alle Alkoholdehydrogenasen geeignet, die die Reduktion von Aldehyden zu Alkanolen katalysieren können. Diese Enzyme werden auch als AlkohoI:NAD-Oxidoreduktasen charakterisiert. Mit Alkohol-Dehydrogenasen sind insbesondere Alkohol:NAD-Oxidoreduktasen zu verstehen, die mit EC 1.1.1 und insbesondere mit EC 1.1.1.1 oder EC 1.1.1.2 gemäß IUBMB Nomenklatur gekennzeichnet werden.

Weiterhin ist bevorzugt, dass die Alkohol-Dehydrogenase-Aktivität von einer Alkohol-Dehydrogenase ausgeht, die ausgewählt ist aus der Gruppe bestehend aus Pferdeleber-Alkoholdehydrogenase (SEQ ID NO: 10), *Thermoanaerobacter brockii* ADH (SEQ ID NO: 13) und *Clostridium ljungdahlii* AdhE2 (SEQ ID NO: 7) oder Varianten davon.

In einem weiteren bevorzugten Verfahren weist die Alkohol-Dehydrogenase eine der in irgendeiner der SEQ ID NOs: 10, 13 und 7 gezeigten Aminosäuresequenzen auf oder eine Aminosäuresequenz, deren Aminosäurepositionen mindestens zu 15 %, bevorzugt mindestens zu 25 %, weiter bevorzugt zu mindestens 50 % und noch weiter bevorzugt zu mindestens 60 % identisch sind mit irgendeiner der in SEQ ID NOs: 10, 13 und 7 gezeigten Aminosäuresequenzen.

In einem weiteren bevorzugten Verfahren wird die Verbindung mit der Formel (II) erhalten durch Umsetzung einer Verbindung mit der Formel (I) [Crotonyl-CoA] 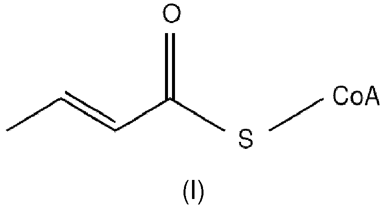 durch Kontaktieren der Verbindung mit der Formel (I) mit einer Vinylacetyl-CoA-Isomerase-Aktivität.

Die Vinylacetyl-CoA-Isomerase-Aktivität geht insbesondere von einer Vinylacetyl-CoA-Isomerase aus. Grundsätzlich sind für die Umsetzung von Crotonyl-CoA zu Vinylacetyl-CoA alle Isomerasen geeignet, die die Isomerisierung von Crotonyl-CoA zu Vinylacetyl-CoA katalysieren können. Insbesondere sind Isomerasen geeignet, die gemäß IUBMB Nomenklatur mit EC 5.3.3.3 oder EC 4.2.1.17 gekennzeichnet werden.

Die Vinylacetyl-CoA-Isomerase ist vorzugsweise ausgewählt aus der Gruppe bestehend aus *Clostridium aminobutyricum* AbfD (SEQ ID NO: 15) und *Streptomyces collinus* ChcB (SEQ ID NO: 18) oder Varianten davon.

In einem weiteren bevorzugten Verfahren weist die Isomerase eine der in irgendeiner der SEQ ID NOs: 15 und 18 gezeigten Aminosäuresequenzen auf oder eine Aminosäuresequenz, deren Aminosäurepositionen mindestens zu 15 %, bevorzugt mindestens zu 25 %, weiter bevorzugt zu mindestens 50 % und noch weiter bevorzugt zu mindestens 60 % identisch sind mit irgendeiner der in SEQ ID NOs: 15 und 18 gezeigten Aminosäuresequenzen.

Insbesondere ist ein Verfahren bevorzugt, wobei das Verfahren in Anwesenheit eines Mikroorganismus durchgeführt wird, der Aldehyd-Dehydrogenase-Aktivität, und optional Alkohol-Dehydrogenase-Aktivität und optional Vinylacetyl-CoA-Isomerase-Aktivität besitzt. Besonders bevorzugt ist ein Verfahren, wobei das Verfahren in Anwesenheit eines Mikroorganismus durchgeführt wird, der Acetaldehyd-Dehydrogenase-Aktivität und Alkohol-Dehydrogenase-Aktivität und optional Vinylacetyl-CoA-Isomerase-Aktivität besitzt. Am meisten ist ein Verfahren bevorzugt, das in Anwesenheit eines Mikroorganismus durchgeführt wird, der Acetaldehyd-Dehydrogenase-Aktivität, Alkohol-Dehydrogenase-Aktivität und Vinylacetyl-CoA-Isomerase-Aktivität besitzt. Insbesondere ist bezüglich der Aldehyd-Dehydrogenase-Aktivität eine acylierende bzw. acetylierende Aldehyd-Dehydrogenase-Aktivität für das erfindungsgemäße Verfahren geeignet.

Die vorliegende Erfindung stellt weiterhin ein Verfahren bereit zur enzymatischen Herstellung einer Verbindung mit der Formel (IV) [Vinylethanol, 3-Buten-1-ol] 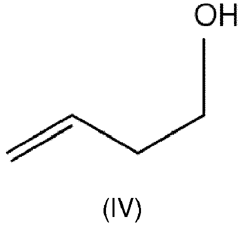 enthaltend die Schritte:
(a) Umsetzung einer Verbindung mit der Formel (II) [Vinylacetyl-CoA, 3-Butenoyl-CoA] 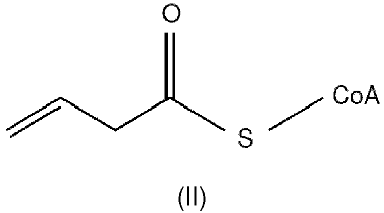 in Anwesenheit einer Aldehyd-Dehydrogenase-Aktivität, wobei eine Verbindung mit der Formel (III) [Vinylacetaldehyd, 3-Butenal] erhalten wird. 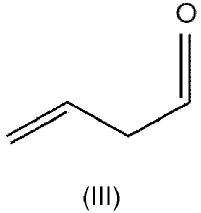
(b) Umsetzung der aus (a) erhaltenen Verbindung mit der Formel (III) in Anwesenheit einer Alkohol-Dehydrogenase-Aktivität, wobei eine Verbindung mit der Formel (IV) [Vinylethanol, 3-Buten-1-ol] erhalten wird 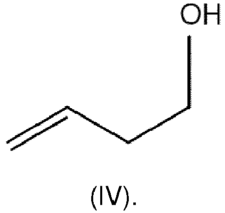 In einer bevorzugten Ausführung des Verfahrens wird die Verbindung mit der Formel (II) erhalten durch Umsetzung einer Verbindung mit der Formel (I) [Crotonyl-CoA] 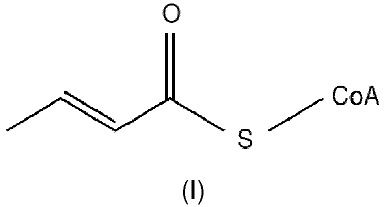 durch Kontaktieren mit einer Vinylacetyl-CoA-Isomerase-Aktivität.

Die Erfindung stellt zudem einen Mikroorganismus bereit, der folgende Merkmale aufweist:
a) der Mikroorganismus exprimiert eine Aldehyd-Dehydrogenase, insbesondere eine acylierende bzw. acetylierende Aldehyd-Dehydrogenase, vorzugsweise ausgewählt aus der Gruppe bestehend aus *Pseudomonas sp.* DmpF (SEQ ID NO: 1), *Comamonas testosteroni* AcDH (SEQ ID NO: 4) und *Clostridium ljungdahlii* AdhE2 (SEQ ID NO: 7), die in der Lage ist, eine Verbindung mit der Formel (III) zu erzeugen;
b) optional, der Mikroorganismus exprimiert eine Alkohol-Dehydrogenase, die in der Lage ist, eine Verbindung mit der Formel (IV) zu erzeugen;
c) optional, der Mikroorganismus exprimiert eine Vinylacetyl-CoA-Isomerase, die in der Lage ist, eine Verbindung mit der Formel (II) zu erzeugen;
d) der Mikroorganismus ist in der Lage, eine Verbindung mit der Formel (III) und/oder (IV) zu erzeugen.

Weiterhin wird eine Zusammensetzung bereitgestellt, enthaltend den oben beschriebenen Mikroorganismus. In einer bevorzugten Ausführungsform der Zusammensetzung enthält diese eine Verbindung mit der Formel (III) und/oder (IV), besonders bevorzugt eine Verbindung mit der Formel (IV).

Die Erfindung stellt zudem die Verwendung des oben beschriebenen Mikroorganismus oder der oben beschriebenen Zusammensetzung bereit zur Herstellung einer Verbindung mit der Formel (III) und/oder (IV), besonders bevorzugt einer Verbindung mit der Formel (IV).

Weiterhin stellt die Erfindung ein Verfahren bereit zur fermentativen Herstellung einer Verbindung mit der Formel (III) und/oder (IV) enthaltend die Schritte:
a) Kultivieren des oben beschriebenen Mikroorganismus in einem geeigneten Medium, wobei eine Fermentationsbrühe erhalten wird, und
b) Anreichern der Verbindung mit der Formel (III) und/oder (IV) in der Fermentationsbrühe aus a).

### Beschreibung der Abbildungen

**Figur 1** zeigt ein Gelbild einer SDS-PAGE zur Überprüfung der Expression der Enzyme CA-AbfD und SC-ChcB. Dabei wurden in den einzelnen Bahnen folgende Proteine aufgetragen:
   M: Proteinmarker, Broad Range (NEB)
   1: CA-AbfD, lösliche Fraktion
   2: CA-AbfD, unlösliche Fraktion
   3: SC-ChcB, lösliche Fraktion
   2: SC-ChcB, unlösliche Fraktion.
**Figur 2** zeigt ein Gelbild einer SDS-PAGE zur Überprüfung der Expression der Enzyme PS-DmpF, CT-AcDH und CL-AdhE2. Dabei wurden in den einzelnen Bahnen folgende Proteine aufgetragen:
   M: Proteinmarker, Broad Range (NEB)
   1: PS-DmpF, lösliche Fraktion
   2: PS-DmpF, unlösliche Fraktion
   3: CT-AcDH, lösliche Fraktion
   4: CT-AcDH, unlösliche Fraktion
   5: CL-AdhE2, lösliche Fraktion
   6: CL-AdhE2, unlösliche Fraktion.
**Figur 3** zeigt ein Gelbild einer SDS-PAGE zur Überprüfung der Expression des Enzyms HL-ADH. Dabei wurden in den einzelnen Bahnen folgende Proteine aufgetragen:
   M: Proteinmarker, Broad Range (NEB)
   1: HL-ADH, lösliche Fraktion
   2: HL-ADH, unlösliche Fraktion.
**Figur 4** zeigt das Ergebnis der photometrischen Aktivitätstests der PS-DmpF gegenüber Butyryl-CoA und Crotonyl-CoA zur Untersuchung einer inhibitorischen Wirkung von Crotonyl-CoA. Die Figur 4 zeigt, dass die Aktivität der PS-DmpF abnimmt, je mehr Crotonyl-CoA im Ansatz enthalten ist. Dies bedeutet, dass Crotonyl-CoA inhibierend auf die Acetaldehyd-Dehydrogenase wirkt.

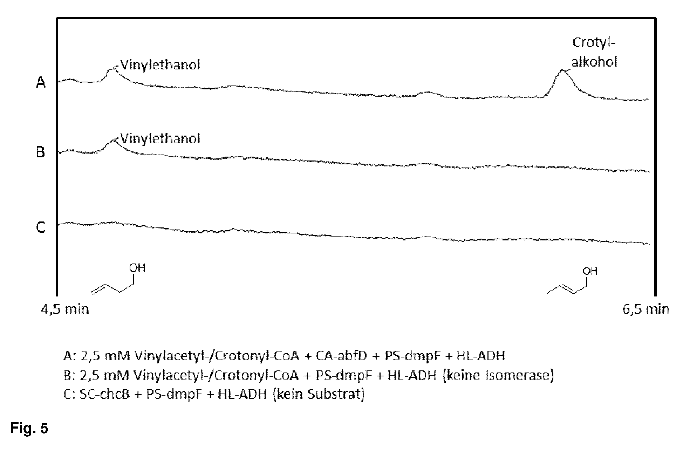

**Figur 5** zeigt einen Vergleich mehrerer Chromatogramme zur Darstellung der Ergebnisse von Reaktionsansätzen, die verschiedene Enzyme enthalten. Die Proben A und B entstammen Reaktionsansätzen mit 2,5 mM Vinylacetyl-CoA/Crotonyl-CoA-Gemisch mit bzw. ohne Isomerasen, sowie den Enzymen PS-DmpF und HL-ADH nach 30 min Inkubationszeit. Die einzelnen Chromatogramme entsprechen folgenden Proben (von oben nach unten): A: Ansatz mit der Isomerase SC-chcb, der Acetaldehyd-Dehydrogenase PS-DmpF und der Alkohol-Dehydrogenase HL-ADH; B: Ansatz mit der Acetaldehyd-Dehydrogenase PS-DmpF und der Alkohol-Dehydrogenase HL-ADH, aber ohne Isomerase; 4: Ansatz mit SC-chcB, PS-DmpF und HL-ADH, ohne Substrat.
**Figur 6** zeigt ein Chromatogramm zur Darstellung des Ergebnisses eines Reaktionsansatzes, welcher Acetaldehyd-Dehydrogenase CT-AcDH enthielt. Die Probe entstammt einem Reaktionsansatz mit 2,5 mM Vinylacetyl-CoA/Crotonyl-CoA-Gemisch nach 120 min Inkubationszeit (Beispiel 12). Das Chromatogramm zeigt deutlich, dass Vinylethanol gebildet wurde.

### Detaillierte Beschreibung der Erfindung

Die vorliegende Erfindung stellt ein Verfahren bereit zur Umsetzung von Vinylacetyl-CoA (3-Butenoyl-CoA) durch Kontaktieren dieser Verbindung mit einer Aldehyd-Dehydrogenase-Aktivität, insbesondere mit einer acylierenden bzw. acetylierenden Aldehyd-Dehydrogenase-Aktivität, wobei Vinylacetaldehyd (3-Butenal) erhalten wird. Bevorzugt ist dabei, dass Vinylacetaldehyd (3-Butenal) weiter umgesetzt zu Vinylethanol (3-Buten-1-ol) durch Kontaktieren von Vinylacetaldehyd (3-Butenal) mit einer Alkohol-Dehydrogenase-Aktivität. Weiterhin ist ein Verfahren bevorzugt, wobei Vinylacetyl-CoA (3-Butenoyl-CoA) erhalten wird durch Umsetzung von Crotonyl-CoA durch Kontaktieren mit einer Vinylacetyl-CoA-Isomerase-Aktivität.

Insbesondere stellt die Erfindung ein Verfahren bereit zur enzymatischen Herstellung von Vinylethanol (3-Buten-1-ol) enthaltend die Schritte:
(a) Umsetzung von Vinylacetyl-CoA (3-Butenoyl-CoA) in Anwesenheit einer Aldehyd-Dehydrogenase-Aktivität, insbesondere mit einer acylierenden bzw. acetylierenden Aldehyd-Dehydrogenase-Aktivität, wobei Vinylacetaldehyd (3-Butenal) erhalten wird;
(b) Umsetzung von aus (a) erhaltenem Vinylacetaldehyd (3-Butenal) in Anwesenheit einer Alkohol-Dehydrogenase-Aktivität, wobei Vinylethanol (3-Buten-1-ol) erhalten wird.

Dabei ist ein Verfahren bevorzugt, wobei Vinylacetyl-CoA (3-Butenoyl-CoA) erhalten wird durch Umsetzung bzw. Isomerisierung von Crotonyl-CoA durch Kontaktieren mit einer Vinylacetyl-CoA-Isomerase-Aktivität.

Die vorliegende Erfindung stellt daher neue Verfahren bereit, mit denen mit Hilfe von Enzymen Vorstufen von 1,3-Butadien, nämlich Vinylacetaldehyd (3-Butenal) und Vinylethanol (3-Buten-1-ol) bereitgestellt werden können. Überraschenderweise wurde festgestellt, dass insbesondere acetylierende Aldehyd-Dehydrogenasen bzw. Acetaldehyd-Dehydrogenasen für die Synthese von Vinylethanol (3-Buten-1-ol) aus Vinylacetyl-CoA (3-Butenoyl-CoA) verwendet werden können.

Als Edukt für das erfindungsgemäße Verfahren wurde Vinylacetyl-CoA (3-Butenoyl-CoA, Formel (II)), das im Gleichgewicht mit Crotonyl-CoA (Formel (I)) vorliegt, gewählt. Die Umwandlung von Crotonyl-CoA (Formel (I)) zu Vinylacetyl-CoA (3-Butenoyl-CoA, Formel (II)) wird von Vinylacetyl-CoA-Isomerasen katalysiert (Reaktionsschritt 1). In den Untersuchungen zur vorliegenden Erfindung konnte gezeigt werden, dass Vinylacetyl-CoA (3-Butenoyl-CoA, Formel (II)) mit Hilfe von Acetaldehyd-Dehydrogenasen (Reaktionsschritt 2) zu Vinylacetaldehyd (3-Butanal, Formel (III)) und Vinylacetaldehyd mit Alkohol-Dehydrogenasen (Reaktionsschritt 3) weiter zu Vinylethanol (3-Buten-1-ol; Formel (IV)) umgesetzt wird. Das folgende Reaktionsschema zeigt die einzelnen Schritte:

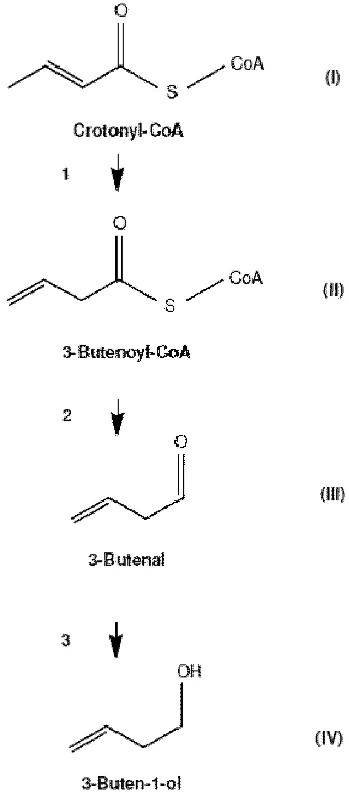

Die Synthese von Vinylethanol (3-Buten-1-ol) ausgehend von einem Gemisch aus Vinylacetyl-CoA und Crotonyl-CoA konnte mit Hilfe diverser Acetaldehyd-Dehydrogenasen und Alkohol-Dehydrogenasen nachwiesen werden.

Mit der vorliegenden Erfindung werden neue Verfahren zur Herstellung von Vorstufen von 1,3-Butadien, nämlich Vinylacetaldehyd (3-Butenal) und Vinylethanol (3-Buten-1-ol), bereitgestellt. In ihren Studien zur vorliegenden Erfindung haben die Erfinder festgestellt, dass Aldehyd-Dehydrogenasen, insbesondere acylierende Aldehyd-Dehydrogenasen für die Reduktion von Vinylacetyl-CoA geeignet sind. So wurden drei verschiedene Acetaldehyd-Dehydrogenasen für die Synthese von Vinylethanol (3-Buten-1-ol) aus Vinylacetyl-CoA (3-Butenoyl-CoA) getestet und die gewünschte Aktivität bezüglich des Substrates Vinylacetyl-CoA bestätigt: *Pseudomonas sp.* DmpF (PS-DmpF), *Comamonas testosteroni* AcDH (CT-AcDH) und *Clostridium Ijungdahlii* AdhE2 (CL-AdhE2).

Nach Klonierung und Expression der für diese Enzyme kodierenden Gene (*Pseudomonas sp. dmpF, Comamonas testosteroni acDH* und *Clostridium Ijungdahlii adhE2)* wurden zunächst Aktivitätstests durchgeführt. Die getesteten Enzyme waren gegenüber dem Substrat Butyryl-CoA aktiv und setzten Butyryl-CoA zu Butanol um. Weiterhin wurde auch beim Einsatz eines Gemisches von Vinylacetyl-CoA und Crotonyl-CoA als Substrat festgestellt, dass diese Enzyme aktiv waren und die CoA-Verbindungen zu den entsprechenden Alkoholen umsetzten. Zudem wurde gezeigt, dass durch Reduktion eines Vinylacetyl-CoA/Crotonyl-CoA-Gemisches mit der CT-AcDH in einigen Fällen ein Gemisch aus Crotylalkohol und Vinylethanol entsteht, und in den meisten Fällen ausschließlich Vinylethanol. Damit zeigt nach den Ergebnissen der Erfinder das Enzym CT-AcDH eindeutig eine Präferenz für die Vinylverbindung. Ansätze mit der CL-AdhE2 ergaben in allen Fällen ein Gemisch aus Vinylethanol und Crotylalkohol, auch wenn keine zusätzliche Alkohol-Dehydrogenase answesend war. Bei der CL-AdhE2 handelt es sich um ein bifunktionales Enzym, das neben der Aldehyd-Dehydrogenase-Aktivität auch eine Alkoholdehydrogenase-Aktivität aufweist. Das Enzym CL-AdhE2 weist keine Selektivität auf.

Mit der CT-AcDH wurde ein Enzym identifiziert, das ein Gemisch aus Vinylacetyl-CoA und Crotonyl-CoA bevorzugt zu Vinylacetaldehyd reduziert.

Die Ergebnisse der vorliegenden Erfindung zeigen weiterhin, dass das Enzym CT-AcDH sowohl NAD als auch NADP als Cofaktor akzeptiert. Bei diesem Enzym handelt es sich nicht um ein bifunktionales Enzym. Wird also das Enzym CT-AcDH bei dem erfindungsgemäßen Verfahren als Aldehyd-Dehydrogenase verwendet, ist die Kopplung mit einer geeigneten Alkohol-Dehydrogenase, vorzugsweise der HL-ADH, notwendig, um Alkohole (hier Vinylethanol) zu synthetisieren.

Weiterhin wurde im Rahmen der Untersuchungen zur vorliegenden Erfindung festgestellt, dass die Acetaldehyd-Dehydrogenase DmpF aus *Pseudomonas* sp. keine Aktivität gegenüber Crotonyl-CoA aufweist. Zudem wurden Untersuchungen angestellt, um zu überprüfen, ob Crotonyl-CoA kein Substrat für dieses Enzym ist oder ob diese Verbindung eine inhibitorische Wirkung auf dieses Enzym besitzt. Dazu wurden Butyryl-CoA, welches von den Acetaldehyd-Dehydrogenasen umgesetzt wird, und Crotonyl-CoA in unterschiedlichen Verhältnissen gemischt und die Aktivität der PS-DmpF gegenüber diesen Substratmischungen untersucht. So wurde gezeigt, dass die Aktivität der PS-DmpF abnimmt, je mehr Crotonyl-CoA im Ansatz enthalten ist. Dies bedeutet, dass Crotonyl-CoA inhibierend auf die Acetaldehyd-Dehydrogenase PS-DmpF wirkt. Da die Folgereaktion zum Vinylacetaldehyd das Vinylacetyl-CoA aus dem Gleichgewicht entzieht, stellt die Hemmung des Enzyms aber kein grundsätzliches Problem dar. Bei Umsetzungen von Vinylacetyl-/Crotonyl-CoA-Gemischen mit der Acetaldehyd-Dehydrogenase PS-DmpF konnte gezeigt werden, dass Vinylethanol und Crotylalkohol gebildet werden. Eine größere Menge an Vinylethanol wurde erzielt, wenn in dem entsprechenden Reaktionsansatz zusätzlich noch Alkohol-Dehydrogenase anwesend war.

Weiterhin haben die Untersuchungen gezeigt, dass aus dem Vinylacetyl-CoA-/Crotonyl-CoA-Gemisch in Abwesenheit von Isomerase und unter Einsatz von Acetaldehyd-Dehydrogenase PS-DmpF Vinylethanol gebildet wird. Dies zeigt die höhere Affinität der PS-DmpF für Vinylacetyl-CoA gegenüber Crotonyl-CoA.

Zusammenfassend wird festgestellt, dass die Versuche zur Synthese von Vinylacetaldehyd mit den Aldehyd-Dehydrogenasen aus *Pseudomonas sp.* PS-DmpF, *Comamonas testosteroni* CT-AcDH und *Clostridium Ijungdahlii* CL-AdhE2 erfolgreich durchgeführt wurden. Diese Enzyme wurden exprimiert und Aktivitätstests mit unterschiedlichen Substraten durchgeführt. Gemäß der Erfindung wurde entdeckt, dass die Aldehyd-Dehydrogenasen Vinylacetyl-CoA zu Vinylacetaldehyd umsetzen. Wird das bifunktionale Enzym CL-AdhE2 verwendet, so wird auch Vinylethanol gebildet. Bei Verwendung der anderen Enyzme ohne eigene Alkohl-Dehydrogenase-Aktivität muss eine separate Alkohol-Dehydrogenase zugefügt werden, um Vinylethanol zu erhalten.

Insbesondere ist das Enzym CT-AcDH geeignet, da es eine Selektivität gegenüber Vinylacetyl-CoA aufweist.

### Definitionen

### Aldehyd-Dehydrogenase-Aktivität

Wie bereits erläutert, basiert die Aldehyd-Dehydrogenase-Aktivität, die gemäß der Erfindung zur Umsetzung von Vinylacetyl-CoA zu Vinylacetaldehyd verwendet wird, auf Aldehyd-Dehydrogenasen, insbesondere auf acylierende bzw. acetylierende Aldehyd-Dehydrogenasen. Die Begriffe "acylierend" und "acetylierend" werden synonym verwendet. Eine weitere synonyme Bezeichnung für diese Enzyme ist "acylierende Acetaldehyd-Dehydrogenase". Acylierende bzw. acetylierende Aldehyd-Dehydrogenasen sind Dehydrogenasen, die die Umwandlung von Aldehyden (wie z.B. Acetaldehyd) in das korrespondierende Acyl-CoA (z.B. Acetyl-CoA) und umgekehrt katalysieren. Die Reduktion von Acetyl-CoA zu Acetaldehyd bzw. die Oxidation von Acetaldehyd zu Acetyl-CoA kann wie folgt dargestellt werden:

Acetyl-CoA + NADH + H⁺ <-> CH₃CHO + NAD⁺ + CoA

Die Begriffe "acylierend" bzw. "acetylierend" bedeuten, dass bei der Oxidation von Aldehyden bzw. Acetaldehyd Coenzym A (CoA) acyliert bzw. acetyliert wird. Die acylierenden Aldehyd-Dehydrogenasen werden auch als Acetaldehyd:NAD⁺ Oxidoreduktasen (CoA acetylierend) charakterisiert. Die acylierenden Aldehyd-Dehydrogenasen werden gemäß IUBMB Nomenklatur mit EC 1.2.1.10 bezeichnet. Acylierende Aldehyd-Dehydrogenasen katalysieren nicht nur die Umsetzung von Acetyl-CoA zu Acetaldehyd und umgekehrt, sondern auch die Umsetzung von entsprechenden Substraten mit höheren Acylresten wie z.B. n-Propionyl-CoA zu n-Propanal, von n-Butyryl-CoA zu n-Butanal und gemäß der Erfindung auch von Vinylacetyl-CoA zu Vinylacetaldehyd.

Insbesondere können gemäß der vorliegenden Erfindung Acetaldehyd-Dehydrogenasen verwendet werden, die ausgewählt sind aus der Gruppe bestehend aus *Pseudomonas sp.* DmpF (SEQ ID NO: 1), *Comamonas testosteroni* AcDH (SEQ ID NO: 4) und *Clostridium Ijungdahlii* AdhE2 (SEQ ID NO: 7). Weiterhin können Varianten dieser Acetaldehyd-Dehydrogenasen verwendet werden, die eine in irgend einem der SEQ ID NOs: 1, 4 und 7 gezeigte Aminosäuresequenz aufweisen oder eine Aminosäuresequenz, deren Aminosäurepositionen mindestens zu 15 %, bevorzugt mindestens zu 25 %, weiter bevorzugt zu mindestens 50 % und noch weiter bevorzugt mindestens zu 60 % identisch sind mit irgendeiner der in SEQ ID NOs: 1, 4 und 7 gezeigten Aminosäuresequenzen.

Bei den vorgenannten Enzymen *Pseudomonas sp.* DmpF, *Comamonas testosteroni* AcDH und *Clostridium Ijungdahlii* AdhE2 handelt es sich um Acetaldehyd-Dehydrogenasen (acylierend), das heißt diese Enzyme katalysieren die Umwandlung von Acetaldehyd zu Acetyl-CoA und umgekehrt. Diese Enzyme sind gemäß der vorliegenden Erfindung auch in der Lage, die Umwandlung von Vinylacetyl-CoA zu Vinylacetaldehyd zu katalysieren. Die vorliegende Erfindung ist aber nicht auf die genannten Enzyme beschränkt, sondern umfasst die Verwendung aller Enzyme, die die Umwandlung von Vinylacetyl-CoA zu Vinylacetaldehyd zu katalysieren vermögen.

Außer der beschriebenen acylierenden Aldehyd-Dehydrogenase-Aktivität können einige Enzyme weitere Aktivitäten aufweisen wie z.B. die Umwandlung des Aldehyds in das entsprechende Alkanol. So handelt es sich bei dem Enzym *Clostridium Ijungdahlii* AdhE2 (SEQ ID NO: 7) um ein bifunktionales Enzym und weist neben der acylierenden Aldehyd-Dehydrogenase-Aktivität (Acetaldehyd:NAD⁺ Oxidoreduktase (CoA acetylierend); EC 1.2.1.10) auch eine Alkoholdehydrogenase-Aktivität auf (Alkohol:NAD⁺ Oxidoreduktase, EC 1.1.1.1). Daher kann bei Einsatz dieses Enzyms ein Acyl-CoA, insbesondere Vinylacetyl-CoA, in den entsprechenden Aldehyd, insbesondere Vinylacetaldehyd, und anschließend in den entsprechenden Alkohol, insbesondere Vinylethanol, umgesetzt werden.

### Coenzym A

Coenzym A (auch Koenzym A, kurz CoA oder CoASH) ist ein Coenzym, das zur "Aktivierung" von Alkansäuren und deren Derivaten dient und am Energiestoffwechsel beteiligt ist. Coenzym A entsteht im Organismus und erfüllt im Stoffwechsel wichtige Aufgaben bei der Aktivierung, Umwandlung und Übertragung von Acyl-Gruppen. Es weist eine Thiol-Gruppe (SH-Gruppe) auf, die zur Bildung von Thioestern in der Lage ist. Diese übertragen die Acyl-Reste bereitwillig auf Nucleophile. Daher werden die Thioester auch aktivierte Säure-Derivate und die Bindung der Acyl-Gruppe an das CoA auch als energiereich bezeichnet.

### Alkohol-Dehydrogenasen

Die Alkohol-Dehydrogenase-Aktivität basiert auf Alkohol-Dehydrogenasen (ADH). Alkohol-Dehydrogenasen sind eine Gruppe von Dehydrogenase-Enzymen, die in vielen Organismen vorkommen und die Umwandlung von Alkoholen einerseits und Aldehyden oder Ketonen andererseits unter Reduktion von Nikotinamid-Adenin-Dinukleotid (NAD⁺ zu NADH) katalysieren. Beispiele hierfür sind der letzte Schritt der alkoholischen Gärung durch Hefe, bei der Acetaldehyd zu Ethanol umgewandelt wird als auch die umgekehrte Variante (Ethanol zu Acetaldehyd), die im menschlichen Körper im Rahmen des Alkoholabbaus stattfindet.

Die Reduktion von Alkanalen zu Alkanolen bzw. die Oxidation von Alkanolen zu Alkanalen kann wie folgt dargestellt werden:

R-CHO + NADH + H⁺ <-> R-CH₂OH + NAD⁺,

wobei R einen Alkylrest darstellt.

Alkoholdehydrogenasen stellen eine seit Jahrzehnten in der Biochemie im Zusammenhang mit brauereitechnischen Fermentationsprozessen stark beachtete und biotechnologisch hochrelevante Enzymklasse dar, die verschiedene Gruppen von Isoformen umfasst. So existieren membrangebundene, flavinabhängige Alkoholdehydrogenasen vom *Pseudomonas putida* GP01 AIkJ-Typ, die Flavocofaktoren statt NAD(P)⁺ verwenden. Eine weitere Gruppe umfasst eisenhaltige, gegenüber Sauerstoff empfindliche Alkoholdehydrogenasen, die in Bakterien und in inaktiver Form in Hefe gefunden werden. Eine andere Gruppe umfasst NAD(P)⁺-abhängige Alkoholdehydrogenasen, unter ihnen zinkhaltige Alkoholdehydrogenasen, die im aktiven Zentrum u.a. ein durch Cysteinreste koordinativ gebundenes Zinkatom aufweist, das das Alkoholsubstrat fixiert. In einer bevorzugten Ausführungsform wird unter dem Begriff "Alkoholdehydrogenase", wie hierin verwendet, ein Enzym verstanden, das einen Aldehyd bzw. Keton zu dem entsprechenden primären bzw. sekundären Alkohol reduziert. Bevorzugt handelt es sich bei der Alkoholdehydrogenase im erfindungsgemäßen Verfahren um eine NAD(P)⁺-abhängige Alkoholdehydrogenase, d.h. eine Alkoholdehydrogenase, die NAD(P)⁺ als Cofaktor zur Oxidation des Alkohols bzw. NAD(P)H zur Reduktion des entsprechenden Aldehyds bzw. Ketons verwendet. Bei der Alkoholdehydrogenase handelt es sich weiter bevorzugt um eine NAD(P)⁺-abhängige, zinkhaltige Alkoholdehydrogenase. Insbesondere bezeichnet der Begriff "NAD(P)⁺-abhängige Al-koholdehydrogenase", wie hierin verwendet, eine Alkoholdehydrogenase, die NAD⁺- und/oder NADP⁺-abhängig ist.

Mit Alkohol-Dehydrogenasen sind insbesondere AlkohoI:NAD-Oxidoreduktasen zu verstehen, die mit EC 1.1.1 und insbesondere mit EC 1.1.1.1 oder EC 1.1.1.2 gemäß IUBMB Nomenklatur gekennzeichnet werden.

Insbesondere können gemäß der vorliegenden Erfindung Alkohol-Dehydrogenasen verwendet werden, die ausgewählt sind aus der Gruppe bestehend aus Pferdeleber-Alkoholdehydrogenase (SEQ ID NO: 10), *Thermoanaerobacter brockii* ADH (SEQ ID NO: 13) und *Clostridium Ijungdahlii* AdhE2 (SEQ ID NO: 7). Weiterhin können Varianten dieser Alkohol-Dehydrogenasen verwendet werden, die eine in irgend einem der SEQ ID Nos: 10, 13 und 7 gezeigte Aminosäuresequenz aufweisen oder eine Aminosäuresequenz, deren Aminosäurepositionen mindestens zu 15 %, bevorzugt mindestens zu 25 %, weiter bevorzugt zu mindestens 50 % und noch weiter bevorzugt zu mindestens 60 % identisch sind mit irgendeiner der in SEQ ID NOs: 10, 13 oder 7 gezeigten Aminosäuresequenzen.

Außer der beschriebenen Alkohol-Dehydrogenase-Aktivität können Enzyme weitere Aktivitäten aufweisen wie z.B. die Umwandlung von Acyl-CoA in den entsprechenden Aldehyd. So handelt es sich bei dem Enzym *Clostridium Ijungdahlii* AdhE2 (SEQ ID NO: 7) um ein bifunktionales Enzym, das wie oben bereits erläutert, neben der Alkoholdehydrogenase-Aktivität (Alkohol:NAD⁺ Oxidoreduktase, EC 1.1.1.1) auch eine acylierende Aldehyd-Dehydrogenase-Aktivität (Acetaldehyd:NAD⁺ Oxidoreduktase (CoA acetylierend); EC 1.2.1.10) aufweist. Daher kann bei Einsatz dieses Enzyms ein Acyl-CoA (insbesondere Vinylacetyl-CoA) in den entsprechenden Aldehyd (insbesondere Vinylacetaldehyd) und in den entsprechenden Alkohol (insbesondere Vinylethanol) umgesetzt werden.

### Vinylacetyl-CoA-Isomerase

Die Vinylacetyl-CoA-Isomerase-Aktivität basiert auf Vinylacetyl-CoA-Isomerasen. Unter Vinylacetyl-CoA-Isomerasen sind solche Enzyme zu verstehen, die mit EC 5.3.3.3 oder EC 4.2.1.17 gemäß IUBMB Nomenklatur bezeichnet werden. Vinylacetyl-CoA-Isomerasen sind Isomerasen, die die Umwandlung von Crotonyl-CoA zu Vinylacetyl-CoA katalysieren, sowie die Rückreaktion.

Gemäß der vorliegenden Erfindung können insbesondere Vinylacetyl-CoA-Isomerasen verwendet werden, die ausgewählt sind aus der Gruppe bestehend aus *Clostridium aminobutyricum* AbfD SEQ ID NO: 15) und *Streptomyces collinus* ChcB (SEQ ID NO: 18). Es können zudem Varianten von Vinylacetyl-CoA-Isomerasen verwendet werden, die eine der in SEQ ID NOs: 15 oder 18 gezeigten Aminosäuresequenz aufweisen oder eine Aminosäuresequenz, deren Aminosäurepositionen mindestens zu 15 %, bevorzugt mindestens zu 25 %, weiter bevorzugt zu mindestens 50 % und noch weiter bevorzugt zu mindestens zu 60 % identisch mindestens sind mit irgendeiner der in SEQ ID NOs: 15 oder 18 gezeigten Aminosäuresequenzen.

### Varianten

Wie bereits oben erläutert, können in dem erfindungsgemäßen Verfahren jegliche Enzyme verwendet werden, die die entsprechende katalytische Aktivität für den jeweiligen Reaktionsschritt aufweisen. In bevorzugten Ausführungsformen des erfindungsgemäßen Verfahrens werden jeweils Varianten der oben genannten Enzyme (Aldehyd-Dehydrogenasen, Alkohol-Dehydrogenasen, Vinylacetyl-CoA-Isomerasen) verwendet, deren Aminosäurepositionen mindestens zu 15 %, 25 %, 50 %, 60%, 70%, 75%, 80%, 85%, 90%, 95%, 98% oder mindestens zu 99% identisch zu den oben beschriebenen Aminosäuresequenzen SEQ ID NOs: 1, 4, 7, 10, 13, 15 oder 18 sind, d.h. wobei mindestens 15 %, 25 %, 50 %, 60%, 70%, 75%, 80%, 85%, 90%, 95%, 98% oder mindestens 99% der Aminosäurepositionen identisch zu jenen der oben beschriebenen Aminosäuresequenzen SEQ ID NOs: 1, 4, 7, 10, 13, 15 oder 18 sind. Die prozentuale Identität wird vorzugsweise über die gesamte Länge der Aminosäure oder Nukleinsäureregion berechnet. Eine Reihe von Programmen, die auf einer Vielzahl von Algorithmen beruhen, steht dem Fachmann für den Sequenzvergleich zur Verfügung. In diesem Zusammenhang ergeben die Algorithmen von Needleman und Wunsch oder Smith und Waterman besonders verlässliche Ergebnisse. Für das Alignment der Sequenzen stehen das Programm PileUp (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970)) und Smith and Waterman (Adv. Appl. Math. 2; 482-489 (1981))], die zum Softwarepaket GCG gehören [Genetics Computer Group, 575 Science Drive, Madison, Wisconsin, USA 53711 (1991)] zur Verfügung. Die oben aufgeführten Prozentwerte für die Sequenzidentität werden vorzugsweise mit dem Programm GAP über die gesamte Sequenzregion berechnet.

Gegebenenfalls werden konservative Aminosäureaustausche bevorzugt. Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Im Rahmen der Erfindung werden zur Erzeugung von Mikroorganismen, die die gewünschten und benötigten Enzyme als Polypeptide erzeugen und damit die entsprechenden Enzymaktivitäten bereitstellen, auch Polynukleotide verwendet, die für die zur Herstellung der Verbindung mit der Formel (III) und/oder (IV) benötigten Enzyme kodieren. Diese Polynukleotide weisen zur Expression der Aldehyddehydrogenase eine Nukleotidsequenz ausgewählt aus SEQ ID NOs: 2, 3, 5, 6, 8 und 9 auf, zur Epression der Alkoholdehydrogenase eine Nukleotidsequenz ausgewählt aus SEQ ID NOs 11, 12 und 14 auf, und zur Expression der Vinylacetyl-CoA-Isomerase eine Nukleotidsequenz ausgewählt aus SEQ ID NOs: 16, 17, 19 und 20 auf. Weiterhin können Polynukleotide eingesetzt werden, die mit der entsprechenden zu SEQ ID NOs: 2, 3, 5, 6, 8, 9, 11, 12, 14, 16, 17, 19 oder 20, bevorzugt der Kodierregion von SEQ ID NOs: 2, 3, 5, 6, 8, 9, 11, 12, 14, 16, 17, 19 oder 20 komplementären Nukleotidsequenz unter stringenten Bedingungen hybridisieren und für ein entsprechendes Polypeptid mit einer Sequenz ausgewählt aus SEQ ID NOs: 1, 4, 7, 10, 13, 15 und 18, welches ein Bestandteil der jeweiligen Enzyme ist, kodieren. In Tabelle 1 und 1a wird die Zuordnung der SEQ ID NOs zu den Enzymen bzw. deren Gene aufgezeigt.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NOs: 2, 3, 5, 6, 8, 9, 11, 12, 14, 16, 17, 19 oder 20 komplementäre Nukleotidsequenz und die Zielsequenz, d.h. die mit der Sonde behandelten beziehungsweise identifizierten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion wird bevorzugt ein Puffer entsprechend 5x SSC-Puffer bei einer Temperatur von ca. 50°C - 68°C eingesetzt. Dabei können Sonden auch mit Polynukleotiden hybridisieren, die weniger als 70% Identität zur Nukleotidsequenz der eingesetzten Sonde aufweisen. Solche Hybride sind weniger stabil und werden durch Waschen unter stringenten Bedingungen entfernt. Dies kann durch Senken der Salzkonzentration auf 2x SSC oder 1 x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 70%, mindestens 80%, mindestens 90%, mindestens 92%, mindestens 94%, mindestens 96%, mindestens 97%, mindestens 98% oder mindestens 99%, gegebenenfalls 100% Identität zur Sequenz beziehungsweise komplementären Sequenz der eingesetzten Sonde besitzen und für Polypeptid, welches ein Bestandteil eines der oben genannten Enzyme ist, kodieren. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

### Art der Bereitstellung der Enzym-Aktivitäten

In dem erfindungsgemäßen Verfahren werden Aldehyd-Dehydrogenasen zur Umsetzung des Substrates Vinylacetyl-CoA zum Vinylacetaldehyd und optional Alkohol-Deyhdrogenasen zur Umsetzung des Vinylacetaldehyds zu Vinylalkohol, sowie optional Isomerasen zur Isomerisierung von Crotonyl-CoA zum Vinylacetyl-CoA verwendet.

Dabei können die Reaktionen in Mikroorganismen ablaufen, die die entsprechenden Enzyme exprimieren und auch das Substrat Vinylacetyl-CoA oder die Vorstufe Crotonyl-CoA aus den Mikroorganismen eigenen oder in diese rekombinant hinzugefügten Biosynthesewege im Rahmen eines Fermentationsverfahrens erzeugt werden. Das gewünschte Produkt Vinylacetaldehyd bzw. Vinylethanol wird entweder von der Zelle in das Medium ausgeschleust, aus dem es abgeerntet und aufgereinigt werden kann, oder es wird nach Abschluss oder kontinuierlich während des Fermentationsverfahren durch Zellaufschluss aus den Zellen gewonnen und aufgereinigt.

Gemäß einem bevorzugten Verfahren der vorliegenden Erfindung erfolgt die Produktion der Verbindung mit der Formel (III) und/oder (IV) (Vinylethanol) mit Hilfe eines fermentativen Verfahrens. Damit stellt die Erfindung ein Verfahren bereit zur fermentativen Herstellung einer Verbindung mit der Formel (III) und/oder (IV) enthaltend die Schritte:
a) Kultivieren des oben beschriebenen Mikroorganismus in einem geeignete Medium, wobei eine Fermentationsbrühe erhalten wird, und
b) Anreichern der Verbindung mit der Formel (III) und/oder (IV) in der Fermentationsbrühe aus a).

Bei den Verfahren können unterschiedliche Mikroorganismen eingesetzt werden, die entsprechend unterschiedliche Rohstoffe für die Fermentation nutzen können. Das Medium, in dem der Mikroorganismus kultiviert wird, kann als fermentierbaren Rohstoff Synthesegas, Erdgas, aus der Petrochemie stammende Rohstoffe wie Kohlenwasserstoffe, Glycerin, CO₂/H₂O, Hausmüll, Getreide, Holzstoff, Lignozellulose, Hemizellulose, Saccharide, Monosaccharide, Disaccharide, Cellulose, Lignin, Triglyceriden wie Glycerin und Fettsäuren, landwirtschaftliche Abfälle und andere Rohstoffe enthalten. In einem bevorzugten Verfahren werden Mikroorganismen in einem Medium mit einer fermentierbaren Kohlenstoffquelle, insbesondere Saccharide wie Glukose, Saccharose, oder Saccharide aus Zellulose oder Hemicellulose, kultiviert. Der Mikroorganismus exprimiert dabei alle Enzyme des Syntheseweges, die zur Umwandlung der Kohlenstoffquelle bis zur Verbindung mit der Formel (III) und/oder (IV) notwendig sind.

Ein zentraler Metabolit der bei der Glykolyse von Kohlenhydraten als Kohlenstoffquelle anfällt ist Pyruvat. Pyruvat wird durch oxidative Decarboxylierung zum Acetyl-CoA umgesetzt.

Die Glykolyse ist der wichtigste Abbauweg der Kohlenhydrate im Stoffwechsel und findet im Cytoplasma jeder Zelle statt. In Prokaryoten und in überwiegend anaerob arbeitenden Zellen oder Geweben (Skelettmuskel) von Eukaryoten wird Pyruvat anaerob zu Milchsäure oder wie bei vielen Hefen zu Ethanol und Kohlendioxid (CO₂) verstoffwechselt. Aerob arbeitende Gewebe (Prototyp: Herzmuskel) bauen den "C3-Körper" Pyruvat zu Acetyl-CoA und Kohlenstoffdioxid ab und das Acetyl-CoA weiter im Citratzyklus zu CO₂ und Wasserstoff. Der Wasserstoff wird dabei an die Wasserstoffüberträger Nicotinamidadenindinukleotid (NAD) und Flavinadenindinukleotid (FAD) gebunden (NADH bzw. FADH₂). Die Energie aus der Oxidation des Wasserstoffs in der Atmungskette der Mitochondrien wird zur Synthese von ATP genutzt (oxidative Phosphorylierung). Der erste Schritt der Glykolyse ist die Phosphorylierung von Glukose zu Glucose-6-phosphat (G6P). In Abhängigkeit vom Zelltyp wird diese Reaktion durch das Enzym Hexokinase oder Glucokinase katalysiert. Bei der Phosphorylierung wird 1 ATP verbraucht. Glucose-6-phosphat wird dann von der Phosphohexose-Isomerase in Fructose-6-phosphat (F6P) umgebaut. Danach wird Fruktose-6-phosphat unter Einwirkung des Schlüsselenzyms der Glykolyse Phosphofructokinase mit einem Molekül ATP zu Fruktose-1,6-bisphosphat (1,6-FBP) phosphoryliert, wobei aus ATP ADP gebildet wird. Die zweite Phosphatgruppe erlaubt nun die Spaltung des Glukoserings durch Aldolase in Dihydroxyacetonphosphat (DHAP) (phosphorylierte Keto-Triose) und Glycerinaldehyd-3-phosphat (3-GAP) (phosphorylierte Aldo-Triose). Dihydroxyacetonphosphat wird von der Triosephosphatisomerase (TIM) in Glycerinaldehyd-3-phosphat umgewandelt. Jedes der beiden resultierenden Glycerinaldehyd-3-phosphat-Moleküle wird dann durch NAD⁺ und Glycerinaldehyd-3-phosphat-Dehydrogenase (GAPDH) zu 1,3-Bisphosphoglycerat (1,3-BPG) oxidiert. Dabei entsteht in einer Zwischenstufe ein Thioester. In chemischem Sinne findet mit dieser Reaktion die Oxidation der Carbonylgruppe zur Carboxylgruppe, d.h. der Übergang vom Zucker(-Phosphat) zum Carbonsäure(-Phosphat) statt. Im nächsten Schritt erzeugt die Phosphoglyceratkinase je ein Molekül ATP bei der Umwandlung von 1,3-Bisphosphoglycerat zu 3-Phosphoglycerat durch Übertragung eines Phosphatrests auf ADP. Die Phosphoglyceromutase katalysiert dann die Umwandlung von 3-Phosphoglycerat zu 2-Phosphoglycerat, woraus schließlich mit Hilfe der Enolase Phosphoenolypyruvat wird. Dieses wird schließlich in der Pyruvatkinasereaktion unter Erzeugung eines weiteren ATP zu Pyruvat. Der Pyruvatdehydrogenase-Komplex setzt das Pyruvat unter Abspaltung von CO₂ zu Acetyl-CoA um. Dabei wird ein HS-CoA gebunden und NAD⁺ zu NADH umgesetzt.

Bei der Fermentation wird gemäß der vorliegenden Erfindung ein Mikroorganismus eingesetzt, der entweder von Natur aus oder durch rekombinante Techniken mit Enzymen ausgestattet ist, die das Acetyl-CoA weiter zum Acetoacetyl-CoA danach zum 3-Hydroxybutyryl-CoA und schließlich zum Crotonyl-CoA umsetzen.

Die Umsetzung von Acetyl-CoA zum Acetoacetyl-CoA erfolgt durch eine Acetyl-CoA C-Acetyltransferase (EC Nummer 2.3.1.9). Dieses Enzym wird von dem Gen *atoB* kodiert. Es sind auch andere Gene bekannt, die ein geeignetes Enzym kodieren. Acetoacetyl-CoA ist ein natürlich vorkommendes metabolisches Zwischenprodukt, welches in den meisten Wirtszellen durch Kondensation von zwei Acetyl-CoA-Körpern gebildet wird.

Die Umsetzung von Acetoacetyl-CoA zum 3-Hydroxybutanoyl-CoA erfolgt bevorzugt durch eine 3-Hydroxybutanoyl-CoA Dehydrogenase (EC 1.1.1.35, EC 1.1.1.36, EC 1.1.1.157 und EC 1.1.100). Das Gen *phaB* kodiert ein geeignetes Enzym.

Die Umsetzung von 3-Hydroxybutanoyl-CoA zum Crotonyl-CoA erfolgt bevorzugt durch eine 3-Hydroxybutanoyl-CoA Dehydratase (EC Nummer 4.2.1.55, EC 4.2.1.17 oder EC 4.2.1.119). Das Gen *phaJ* kodiert ein geeignetes Enzym.

Im Fermentationsverfahren gemäß der Erfindung wird das Crotonyl-CoA von der Vinylacetyl-CoA-Isomerase zum Vinylacetyl-CoA umgesetzt. Das Vinylacetyl-CoA wiederum wird von der Aldehyd-Dehydrogenase zum Vinylacetaldehyd umgesetzt. Weiterhin kann gemäß der Erfindung das Vinylacetaldehyd von der Alkoholdehydrogenase zum Vinylethanol umgesetzt werden.

Somit wird eine vollständige fermentative Umsetzung, vorzugsweise ausgehend von einer fermentierbaren Kohlenstoffquelle (vorzugsweise Kohlenhydrate), bis hin zum Vinylacetaldehyd bzw. zum Vinylethanol mit Hilfe eines mit entsprechenden Enzymen ausgestatteten Mikroorganismus bereitgestellt.

Geeignete Wirtszellen für die Fermentation können sein Prokaryoten der Gattung *Escherichia* wie *Escherichia coli;* aus der Gattung *Clostridium* wie *Clostridium ljungdahlii, Clostridium autoethanogenum* oder *Clostridium kluyveri;* aus der Gattung *Corynebacterium* wie *Corynebacterium glutamicum*; aus der Gattung *Cupriavidus* wie *Cupriavidus necator* oder *Cupriavidus metallidurans*; aus der Gattung *Pseudomonas* wie *Pseudomonas fluorescens, Pseudomonas putida* oder *Pseudomonas oleavorans*; aus der Gattung Delftia wie *Delftia acidovorans*; aus der Gattung *Bacillus* wie *Bacillus subtillis*; aus der Gattung *Lactobacillus* wie *Lactobacillus delbrueckii;* oder aus der Gattung *Lactococcus* wie *Lactococcus lactis.* Der Wirtsorganismus kann auch ein Eurkaryot sein aus der Gattung *Aspergillus* wie *Aspergillus niger*; aus der Gattung *Saccharomyces* wie *Saccharomyces cerevisiae*; aus der Gattung *Pichia* wie *Pichia pastoris*; aus der Gattung *Yarrowia* wie *Yarrowia lipolytica*; aus der Gattung *Issatchenkia* wie *Issathenkia orientalis*; aus der Gattung *Debaryomyces* wie *Debaryomyces hansenii*; aus der Gattung *Arxula* wie *Arxula adenoinivorans*; oder aus der Gattung *Kluyveromyces* wie *Kluyveromyces lactis.*

Die Fermentationsverfahren können anaerob oder aerob durchgeführt werden.

Ein effizienter Abbaustoffwechsel ausgehend von Rohglycerin aus der Produktion von Biodiesel wurde in verschiedenen Mikroorganismen wie *Escherichia coli, Cupriavidus necator, Pseudomonas oleavorans, Pseudomonas putida* und *Yarrowia lipolytica* gezeigt.

Ein effizienter Abbaustoffwechsel ausgehend von aus Lignocellulose stammender Levulinsäure wurde in verschiedenen Mikroorganismen wie *Cupriavidus necator* und *Pseudomonas putida* gezeigt.

Ein effizienter Abbaustoffwechsel ausgehend von Lignin stammenden aromatischen Verbindungen wie Benzoat-Analogen wurde in verschiedenen Mikroorganismen wie *Pseudomonas putida, Cupriavidus necator* gezeigt.

Ein effizienter Abbaustoffwechsel ausgehend von landwirtschaftlichen Abfällen wie Olivenmühlenabwässer wurde in verschiedenen Mikroorganismen einschließlich *Yarrowia lipolytica* gezeigt.

Die effiziente Verwendung von fermentierbaren Sacchariden wie Monosacchariden und Disacchariden, die aus cellulosischen, hemicellulosischen Quellen, aus Zuckerrohr und Rübenmelassen, Cassava, Getreide und anderen landwirtschaftlichen Quellen stammen, wurden für verschiedene Mikroorganismen wie *Escherichia coli, Corynebacterium glutamicum* und *Lactobacillus delbrueckii* und *Lactococcus lactis* gezeigt.

Die effiziente Verwendung von Furfural, welches aus einer Vielzahl von landwirtschaftlichen Lignocellulose-Quellen entstammt, wurde für *Cupriavidus necator* beschrieben.

In einigen Ausführungsformen ist oder stammt der nicht biologische Rohstoff von Erdgas, Synthesegas, CO₂/H₂, Methanol, Ethanol, von nicht flüchtigen Stoffen oder aus Cyclohexan-Oxidations-Verfahren.

Die effiziente Verwendung von Methanol wurde für *Pichia pastoris* gezeigt.

Der effiziente Abbaustoffwechsel ausgehend von Ethanol wurde für *Clostridium kluyveri* gezeigt.

Ein effizienter Abbaustoffwechsel ausgehend von CO₂ und H₂, welche von Erdgas oder anderen chemischen oder petrochemischen Quellen stammen können, wurde für *Cupriavidus necator* gezeigt.

Der effiziente Abbaustoffwechsel ausgehend von Synthesegas wurde für zahlreiche Mikroorganismen wie *Clostridium ljungdahlii* und *Clostridium autoethanogenum* gezeigt (Köpke et al., Applied and Environmental Microbiology, 2011, 77(15), 5467-5475).

Der effiziente Abbaustoffwechsel ausgehend von nicht flüchtigen Restabfall-Strömen aus Cyclohexan-Verfahren wurde für zahlreiche Mikroorganismen wie *Delftia acidovorans* und *Cupriavidus necator* gezeigt.

In einem alternativen Verfahren können die für die genannten Reaktionen eingesetzten Enzymaktivitäten bzw. Enzyme dem in einer Lösung vorgelegten Substrat oder den Substraten in Form von enzymatisch aktiven Polypeptiden, Zellen umfassend enzymatisch aktive Polypeptide oder deren Lysate oder Präparationen der Polypeptide in sämtlichen Aufreinigungsstufen, vom kruden Lysat bis zum reinen Polypeptid, zugesetzt werden.

Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden. Zur Aufreinigung kommen chromatographische Verfahren in Frage, wie etwa die affinitätschromatographische Aufreinigung eines mit einer zur Aufreinigung geeigneten Markierung (tag) versehenen rekombinanten Proteins unter Verwendung eines immobilisierten Liganden, vorzugsweise eines Nickelions im Falle einer Histidin-Markierung (z.B. Hexahistidin-Sequenz), von immobilisiertem Glutathion im Falle einer an das Zielprotein fusionierten Glutathion-S-Transferase oder von immobilisierter Maltose im Falle einer Markierung umfassend Maltosebindendes Protein. Die aufgereinigten enzymatisch aktiven Polypeptide können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, etwa durch durch Sulfhydryl-KupplungsChemie (z.B. Kits der Firma Pierce).

In einer bevorzugten Ausführungsform der Erfindung werden die oben beschriebenen Enzymaktivitäten und Enzyme in Form eines Ganzzellkatalysators bereitgestellt, der das entsprechende Enzym aufweist. In einer weiteren Ausführungsform werden sämtliche Enzyme in Form von einem oder mehr als einem Ganzzellkatalysator bereitgestellt, wobei bevorzugt ein Ganzzellkatalysator sämtliche für das erfindungsgemäße Verfahren erforderlichen Enzyme aufweist.

In einer bevorzugten Ausführungsform handelt es sich bei der als Ganzzellkatalysator oder bei der als Expressionssystem verwendeten Zelle um eine prokaryotische, bevorzugt um eine bakterielle Zelle. In einer weiteren bevorzugten Ausführungsform handelt es sich um eine niedere eukaryotische Zelle, bevorzugt um eine Hefezelle. Prokaryotische Zellen umfassen vorzugsweise *Escherichia,* insbesondere besonders *Escherichia coli* und Stämme der Gattung *Pseudomonas* und *Corynebacterium.* Niedere eukaryotische Zellen umfassen vorzugsweise die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.*

Bei den erfindungsgemäß verwendeten Enzymen handelt es sich bevorzugt um rekombinante Enzyme. Unter dem in dieser Anmeldung verwendeten Begriff "rekombinant" wird verstanden, dass das entsprechende für das Enzym kodierende Nukleinsäuremolekül in dem betreffenden Mikroorganismus nicht, nicht in dieser Form bzw. der kodierenden Sequenz oder nicht in der molekulargenetischen Umgebung vorkommt und/oder es unter Verwendung von gentechnischen Methoden hergestellt wurde. Insbesondere spricht man von einem rekombinanten Protein, wenn das entsprechende Polypeptid von einer rekombinanten Nukleinsäure kodiert ist. Dabei kann die Aminosäuresequenz des Polypeptides identisch sein mit der Wildtyp-Sequenz. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, insbesondere die in Sambrook et al., 1989 (Sambrook, J.; Fritsch, E. F. und Maniatis, T. (1989), Molecular cloning: a laboratory manual, 2nd ed., N.Y., Cold Spring Harbor Laboratory, Cold Spring Harbor Laboratory Press, 1989. 1659 p. ISBN 0-87969-309-6), beschriebenen.

Das erfindungsgemäße Verfahren kann sowohl unter Verwendung von isolierten Enzymen als auch unter Verwendung von Ganzzellkatalysatoren ausgeführt werden. In einer bevorzugten Ausführungsform versteht man unter dem Begriff "Ganzzellkatalysator", wie hierin verwendet, eine intakte, lebensfähige und metabolisch aktive Zelle, die die erwünschte(n) enzymatische(n) Aktivität(en) bereitstellt. Der Ganzzellkatalysator kann das zu verstoffwechselnde Substrat, im Falle der vorliegenden Erfindung das Vinylacetyl-CoA, entweder ins Zellinnere transportieren, wo es von genannten Enzymen umgesetzt wird, oder der Ganzzellkatalysator kann das Enzym von Interesse auf seiner Oberfläche präsentieren, wo es gegenüber den genannten im Medium vorliegenden Substraten direkt exponiert ist. In einer bevorzugten Ausführungsform wird für das erfindungsgemäße Verfahren ein Ganzzellkatalysator mit allen erforderlichen Aktivitäten eingesetzt. Die Verwendung eines derartigen Ganzzellkatalysators hat den Vorteil, dass sämtliche Aktivitäten in Form von einem einzelnen Agens eingesetzt werden und es nicht notwendig ist, Enzyme in biologisch aktiver Form großtechnisch aufzubereiten. Dem Fachmann sind geeignete Verfahren zur Konstruktion von Ganzzellkatalysatoren bekannt, insbesondere die Konstruktion von Plasmidsystemen zur Expression von einem oder mehr als einem rekombinanten Protein oder die Integration der für die erforderlichen rekombinanten Protein kodierenden DNA in die chromosomale DNA der verwendenden Wirtszelle.

Eine bevorzugte Variante für die Durchführung des erfindungsgemäßen Verfahrens ist die Verwendung isolierter Enzyme. In einer bevorzugten Ausführungsform bedeutet der Begriff "isoliert", wie hierin verwendet, dass das Enzym in reinerer Form als in seiner natürlichen Quelle und/oder in aufkonzentrierter Form vorliegt. In einer bevorzugten Ausführungsform gilt das Enzym als isoliert, wenn es ein Polypeptidenzym ist und mehr als 60, 70, 80, 90 oder bevorzugt 95 % des Massenproteinanteils der entsprechenden Präparation ausmacht. Dem Fachmann sind zahlreiche Verfahren zur Messung der Masse eines Proteins in einer Lösung bekannt, wie etwa die visuelle Abschätzung anhand der Dicke von entsprechenden Proteinbanden auf SDS-Polyacrylamidgelen, NMR-Spektroskopie oder massenspektrometriebasierte Verfahren.

In einer weiteren Ausgestaltung der vorliegenden Erfindung werden die Enzyme für die Reaktion bereitgestellt, in dem sie von prokaryotischen Zellen exprimiert werden, die rekombinante Plasmide/Vektoren enthalten, die eine oder mehrere der für die Enzyme kodierenden Polynukleotide aufweisen. Unter einem Expressionssystem ist ein System zur rekombinanten Expression der für das Verfahren benötigten Enzyme zu verstehen.

Diese Herstellung der für das erfindungsgemäße Verfahren benötigten Enzym-Aktivitäten bzw. Enzyme erfolgt bevorzugt in mit entsprechenden Nukleinsäuresequenzen oder Vektoren transformierten Mikroorganismen. Geeignete Wirtszellen schließen Zellen von einzelligen Mikroorganismen wie bakterielle Zellen ein. Als Mikroorganismen können diesbezüglich Prokaryonten, wie *E. coli, Bacillus subtilis* oder *Pseudomonas sp.* Ferner können Bakterien der Genera/Spezies *Lactobacillus, Bacillus, Rhodococus, Campylobacter, Caulobacter, Mycobacterium, Streptomyces, Neisseria, Ralstonia, sowie Agrobacterium* für die Expression der für die genannten Enzyme kodierenden Nukleinsäuresequenzen eingesetzt werden. Entsprechende Stämme sind im Stand der Technik verfügbar und können, zumindest teilweise, über die internationalen Hinterlegungsstellen wie die ATCC oder die DMSZ bezogen werden.

Die Klonierung wird nach Verfahren durchgeführt, die dem Fachmann wohlbekannt sind (Sambrook, J.; Fritsch, E. F. und Maniatis, T. (1989), Molecular cloning: a laboratory manual, 2nd ed., N.Y., Cold Spring Harbor Laboratory, Cold Spring Harbor Laboratory Press, 1989. 1659 p. ISBN 0-87969-309-6). Vorzugsweise werden *E. coli-Stämme* für diesen Zweck benutzt, wobei ganz besonders bevorzugt sind: *E. coli* XL1 Blue, NM 522, JM101, JM109, JM105, RR1, DH5[alpha], TOP 10-, HB101, BL21 codon plus, BL21 (DE3) codon plus, BL21, BL21 (DE3), MM294, W3110.

Die oben beschriebene Transformation kann nach bekannten Methoden erfolgen, z.B. durch Calciumphosphat-Copräzipitation, Lipofektion, Elektroporation, PEG/DMSO-Methode, Partikelbeschuss oder virale/bakteriophage Infektion. Die entsprechende Wirtszelle kann die rekombinante Nukleinsäure in extrachromosomaler oder chromosomal integrierter Form enthalten. Transformations-Protokolle sind dem Fachmann bekannt (Chang and Cohen. 1979. High Frequency Transformation of Bacillus subtilis Protoplasts by Plasmid DNA. Mol Gen Genet. 168(1): 111-115; Sambrook, J.; Fritsch, E. F. und Maniatis, T. (1989), Molecular cloning: a laboratory manual, 2nd ed., N.Y., Cold Spring Harbor Laboratory, Cold Spring Harbor Laboratory Press, 1989. 1659 p. ISBN 0-87969-309-6).

Die codierenden Nukleinsäuresequenzen können in herkömmliche Plasmide/Vektoren kloniert und nach Transformation von Mikroorganismen mit solchen Vektoren in Zellkultur exprimiert werden. Als Plasmide oder Vektoren kommen im Prinzip alle dem Fachmann für diesen Zweck zur Verfügung stehende Ausführungsformen in Frage. Derartige Plasmide und Vektoren können z.B. von Studier und Mitarbeiter (Studier, W. F.; Rosenberg A. H.; Dunn J. J.; Dubendroff J. W.; Use of the T7 RNA polymerase to direct expression of cloned genes, Methods Enzymol. 1990, 185, 61-89) oder den Broschüren der Firmen Novagen, Promega, New England Biolabs, Clontech oder Gibco BRL entnommen werden. Weitere bevorzugte Plasmide und Vektoren können gefunden werden in: Glover, D. M. (1985), DNA cloning: a practical approach, Vol. I-III, IRL Press Ltd., Oxford; Rodriguez, R. L. und Denhardt, D. T (eds) (1988), Vectors: a survey of molecular cloning vectors and their uses, 179-204, Butterworth, Stoneham; Goeddel, D. V., Systems for heterologous gene expression, Methods Enzymol. 1990, 185, 3-7; Sambrook et al. 1989 (Sambrook, J.; Fritsch, E. F. und Maniatis, T. (1989), Molecular cloning: a laboratory manual, 2nd ed., N.Y., Cold Spring Harbor Laboratory, Cold Spring Harbor Laboratory Press, 1989. 1659 p. ISBN 0-87969-309-6).

Plasmide, mit denen die in dieser Erfindung beschriebenen Enzyme in den Wirtsorganismus eingeführt werden können, sind: pUC18 (Roche Biochemicals), pKK-177-3H (Roche Biochemicals), pBTac2 (Roche Biochemicals), pKK223-3 (Amersham Pharmacia Biotech), pKK-233-3 (Stratagene) oder pET (Novagen). Geeignete Vektoren sind z.B. auch pET-21a(+) für *E*. *coli,* aber auch andere Expressionsvektoren für prokaryontische Einzeller. In einer besonders bevorzugten Ausführungsform werden die in Plasmide eingeschleusten für die Enzyme kodierenden Nukleinsäuresequenzen darüber hinaus mit einem von dem Vektor bereitgestellten Histidin-Tag fusioniert. Bevorzugt wird die eingeschleuste Nukleinsäuresequenz in pET-Vektoren, vorzugsweise pET-21a(+) oder pET22b kloniert, dass die Transkription unter der Kontrolle des im Vektor vorhandenen IPTG-regulierbaren Promotors steht.

Die Vektoren können neben den üblichen Markern, wie z.B. Antibiotika-Resistenzgenen, weitere funktionelle Nukleotidsequenzen zur Regulation, insbesondere zur Repression oder Induktion der Expression der Gene kodierend für die oben beschriebenen Enzyme und/oder eines Reportergens enthalten. Als Promotoren werden bevorzugt regulierbare schwache Promotoren, wie z.B. der rha-Promotor oder der nmt1-Promotor, oder regulierbare starke Promotoren, wie z.B. der lac-, ara-, lambda-, pL-, T7- oder T3-Promotor, benutzt. Die codierenden DNA-Fragmente müssen in den Vektoren von einem Promotor aus transkribierbar sein.

Die gemäß der Erfindung verwendeten Expressionsvektoren können weitere funktionale Sequenzbereiche, wie z.B. einen Replikationsstartpunkt, Operatoren, oder Terminationssignale enthalten.

### Kofaktorregenerierung

In einem weiteren bevorzugten Verfahren der vorliegenden Erfindung wird ein gekoppeltes enzymatisches Reaktionssystem genutzt, enthaltend eine Kofaktorabhängige enzymatische Umwandlung eines Substrates mit dem entsprechenden erforderlichen Enzym und eine enzymatische Regeneration des Kofaktors (NADH, NAD(P)H). Die enzymatische Regeneration des Kofaktors sollte vorteilhafterweise mit den oben im Zusammenhang mit dem Ganzellkatalysator besprochenen Enzymen vonstatten gehen, sie kann jedoch auch elektrochemisch oder durch chemische Oxidation ohne den Einsatz von Enzymen durchgeführt werden. Als eine bevorzugte Möglichkeit eines enzymatischen oder substratbasierenden Kofaktor-regenerierenden Systems sei NADH-Oxidase und Glucosedehydrogenase genannt.

### Immobilisierung der Enzyme

Für die Anwendung in dem erfindungsgemäßen Verfahren kann das Enzym bzw. können die Enzyme in nativer Form, als homogen aufgereinigte Verbindungen oder als rekombinant hergestelltes Enzym verwendet werden. Weiterhin kann das Enzym auch als Bestandteil eines intakten Gastorganismus eingesetzt werden oder in Verbindung mit der aufgeschlossenen und beliebig hoch aufgereinigten Zellmasse des Wirtsorganismus. Möglich ist ebenfalls die Verwendung der Enzyme in immobilisierter Form (Sharma B. P.; Bailey L. F. und Messing R. A. (1982), Immobilisierte Biomaterialien - Techniken und Anwendungen, Angew. Chem. 94, 836-852). Vorteilhafterweise erfolgt die Immobilisierung durch Lyophilisation (Paradkar, V. M.; Dordick, J. S. (1994), Aqueous-Like Activity of Chymotrypsin Dissolved in Nearly Anhydrous Organic Solvents, J. Am. Chem. Soc. 116, 5009-5010; Mori, T.; Okahata, Y. (1997), A variety of lipid-coated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Die Lyophilisation kann auch in Gegenwart von oberflächenaktiven Substanzen, wie Aerosol®OT, Polyvinylpyrrolidon oder Polyethylenglycol (PEG) oder Brij 52 (Diethylenglycol-mono-cetylether) durchgeführt warden (Kamiya, N.; Okazaki, S.-Y.; Goto, M. (1997), Surfactanthorseradish peroxidase complex catalytically active in anhydrous benzene, Biotechnol. Tech. 11, 375-378). Weiterhin ist die Immobilisierung an Eupergit® geeignet (Röhm) (als Übersicht, siehe: E. Katchalski-Katzir, D. M. Kraemer, J. Mol. Catal. B: Enzym. (2000), 10, 157-176). Eine andere Möglichkeit ist die Immobilisierung an Ni-NTA in Kombination mit dem durch Anhängen eines His-Tags (Hexa-Histidin) veränderten Polypeptids (Petty, K. J. (1996), Metal-chelate affinity chromatography In: Ausubel, F. M. et al. eds. Current Protocols in Molecular Biology, Vol. 2, New York: John Wiley and Sons).

Die Umsetzung der Substrate mit den Enzymen erfolgt, indem die Enzyme in der gewünschten Form (frei, immobilisiert, in Wirtsorganismen oder als Ganzzellkatalysator) in einer wässrigen Lösung mit dem Substrat, ggf. dem Kofaktor oder den Kofaktoren und ggf. die den Kofaktor regenerierenden Mittel kontaktiert werden, wobei die optimalen Temperaturbereiche bzw. die optimalen pH-Werte eingehalten werden. Nach der erfolgten Umsetzung kann das erhaltene Produkt mit den dem Fachmann bekannten Verfahren (Kristallisation, Extraktion, Chromatographie) aus der Reaktionsmischung isoliert werden.

Die vorliegende Erfindung wird anhand der folgenden Beispiele näher erläutert. Diese sind jedoch in keiner Weise als einschränkend zu verstehen.

### Beispiele

### Beispiel 1: Herstellung des gemischten Anhydrids aus Ethylkohlensäure und Vinylacetat

Um die Aktivität von Enzymen hinsichtlich der Umsetzung von Vinylacetyl-CoA zu Vinylacetaldehyd und dann zu Vinylalkohol untersuchen zu können, wurde das Edukt Vinylacetyl-CoA chemisch synthetisiert. Hierzu wurde zunächst ein Vinylacetylethylcarbonat aus Ethylkohlensäure und Vinylacetat synthtesiert. Da es bei dieser Reaktion zu einer Isomerisierung kommt, lag das gewünschte Produkt Vinylacetylethylcarbonat im Gemisch mit Crotylethylcarbonat vor. Der nächste Syntheseschritt zum Vinylacetyl-CoA wird in Beispiel 2 beschrieben.

Zur Herstellung des Vinylacetylethylcarbonat wurden in einem 500 ml Doppelmantelgefäß mit Inertisierung und Flammschutzwanne 40,4 g frisch destilliertes Triethylamin (0,4 mol, 100% Gehalt, 1,0 Equivalent, CAS 121-44-8, 101,19 g/mol C₆H₁₅N) und 35,5 g Vinylessigsäure (0,4 mol, 97,0% Gehalt, 1,0 Equivalent, CAS 625-38-7, 86,089 g/mol, C₄H₆O₂) in 200 ml Diethylether vorgelegt und auf 0°C abgekühlt. Es wurden 44,3 g Ethylchlorformiat (0,4 mol, 98,0 % Gehalt, 1,0 Equivalent, CAS 541-41-3, 108,523 g/mol, C₃H₅ClO₂) in 50 ml Diethylether unter Rühren bei 0°C zugetropft, 1,5 h nachgerührt und anschließend in 30 min auf 20°C erwärmt. Das ausgefallene Triethylaminhydrochlorid wurde über eine Glassinternutsche Pore3 abfiltriert und zweimal mit je 50 ml Diethylether gewaschen. Die vereinigten Filtrate wurden einmal mit 40 ml Natriumhydrogencarbonatlösung und einmal mit 40 ml Wasser gewaschen und über Magnesiumsulfat wasserfrei getrocknet und filtriert. Anschließend wurde der Diethylether am Rotationsverdampfer entfernt, wobei die Badtemperatur max. 40°C bei 900 mbar betrug. 10 ml des Rückstandes wurden bei 1 - 2 mbar in der Halbmikroapparatur fraktioniert destilliert (Siedepunkt 50 - 60°C).

Eine Probe des Rückstandes wurde mittels GC/MS-EI analysiert. Dabei wurde der Erhalt eines Gemisches aus einem Vinylacetylethylcarbonat und einem Crotylethylcarbonat bestätigt.

### Beispiel 2: Herstellung von Vinylacetyl-CoA im Gemisch mit Crotonyl-CoA aus Coenzym A (CoA)

Zur Herstellung des Vinylacetyl-CoA wurde das gemischte Anhydrid aus Beispiel 1 weiter mit dem Trilithiumsalz des Coenzym A umgesetzt.

Dazu wurden 500 mg Trilithiumsalz des Coenzym A (0,59 mmol, 93,0% Gehalt, 1,00 Equivalent, CAS 18439-24-2, 785,335 g/mol, C₂₁H₃₃Li₃N₇O₁₆P₃S; kommerziell erhältlich von Sigma) in 67,5 ml eines 0,1 M Natriumhydrogencarbonat-Puffers (pH-Wert = 8,0) gelöst und mit dem in Beispiel 1 erhaltenen Gemisch des Vinylacetylethylkohlensäureanhydrids und Crotylethylkohlensäureanhydrids (390,1 mg, 1,48 mmol, 60,0% Gehalt, 2,5 Equivalente, 158,152 g/mol, C₇H₁₉O₄) versetzt und für 1 h bei 24°C gerührt. Der Reaktionsfortschritt wurde mit Ellmans Reagenz (5,5'-dithiobis-(2-nitrobenzoic acid); DTNB) überprüft. Die Reaktion wurde durch Zugabe von 8 ml einer 1 M Salzsäure beendet und ein pH-Wert von 3,4 erreicht. Die Reaktionslösung wurde dreimal mit je 90 ml Diethylether extrahiert. Anschließend wurde für 10 min durch die Reaktionslösung ein leichter Stickstoffstrom geleitet, um Etherreste zu entfernen. Die Lösung wurde in einem 250 ml Rundkolben gegeben, in Trockeneis tiefgefroren und über 16 h gefriergetrocknet. Es wurden mehrere Säulen mit 50 g Kieselgel RP 18 endcapped auf einer Glassinternutsche Pore3 gepackt und mit 100ml MeOH gewaschen und anschließend mit 100 ml VE-Wasser (VE-Wasser = vollentsalztes Wasser, CAS Nr. 7732-18-5) von pH 5 äquilibriert. Der Rückstand aus der Gefriertrocknung wurde in 15 ml VE-Wasser pH 5 Wasser gelöst und in drei gleich große Aliquots aufgeteilt. Es wurde jeweils ein Aliquot auf eine Säule aufgetragen. Die drei Säulen wurden mit je 120 ml VE-Wasser pH 5 gewaschen. Die gebildeten CoA-Verbindungen wurden mit jeweils 100 mL 15 % (v/v) Methanol in Wasser eluiert. Die Anwesenheit der CoA-Verbindungen im Eluat wurde mittels Tüpfelprobe auf Kieselgelplatte mit UV-Licht überprüft. Die drei Eluate wurde jeweils bei max. 30°C am Rotationsverdampfer von Methanol befreit und anschließend über 62 h gefriergetrocknet.

Je eine Probe des Rückstandes wurde mittels HPLC UV und MS analysiert. Die drei Rückstände wurde im Gefrierschrank bei -20°C unter Schutzgas trocken gelagert.
Ausbeute: 1. Drittel: ca. 60mg.
Ausbeute: 2. Drittel: ca. 101 mg
Ausbeute: 3. Drittel: ca. 65mg
Gesamtausbeute: 0,226 mg

Die Elutionszeiten der CoA-Verbindungen betrugen in Minuten: 13,85 Crotonyl-CoA, 14,14 Vinylacetyl-CoA. Zur Unterscheidung welches Isomere zuerst eluiert, wurde käufliches Crotonyl-CoA mit der gleichen Analytik eluiert. Dies zeigte, dass der erste Peak Crotonyl-CoA zuzuordnen ist, während der zweite Peak mit der passenden Masse Vinylacetyl-CoA darstellt.

Die erhaltenen CoA-Verbindungen wiesen die typische UV-Absorption bei 260 nm auf, die dazugehörigen MS zeigten eine Masse von 834 m/z bei negativer Ionisierung, von 836 m/z bei positiver Ionisierung, die Crotonyl-CoA bzw. Vinylacetyl-CoA mit 835 g/mol (als Trilithiumsalz 853 g/mol) entspricht.

Die Analyse ergab, dass ein Crotonyl-CoA/Vinylacetly-CoA Gemisch mit einer Reinheit von 80-90% erhalten wurde mit einem Verhältnis von 70:30 (50:50 je nach Charge) Crotonyl-CoA/Vinylacetly-CoA.

### Beispiel 3: Expression der Enzyme

Zur enzymatischen Umsetzung von Vinylacetyl-CoA zu Vinylacetaldehyd (3-Butenal) und Vinylethanol (3-Buten-1-ol) mussten zunächst die ensprechenden Proteine exprimiert werden. Dazu wurden Gensequenzen verschiedener Acetaldehyd-Dehydrogenasen, Alkohol-Dehydrogenasen und Vinylacetyl-CoA-Isomerasen in Expressionsvektoren kloniert und in *E. coli* exprimiert. Eine Übersicht über die einzelnen Enzyme, Sequenzen und die Herkunftsorganismen gibt Tabelle 1. In Tabelle 1a werden die entsprechenden Wildtyp-Nukleotidsequenzen bzw. die Codon-optimierten Nukleotidsequenzen angegeben, die für das jeweilige Enzym kodieren.

**Tabelle 1: Übersicht über die verwendeten Enzyme**

| **Enzym, Abkürzung, IUBMB Nummer** | **Herkunftsorganismus** | **SEQ ID NO: des Enzyms** | **Reaktionsschritt** |
|---|---|---|---|
| Vinylacetyl-CoA-Isomerase, CA AbfD EC 5.3.3.3 | *Clostridium aminobutyricum* | 15 | 1 |
| Vinylacetyl-CoA-Isomerase, SC ChcB EC 4.2.1.17 | *Streptomyces collinus* | 18 | 1 |
| Acetaldehyd-Dehydrogenase, PS DmpF EC 1.2.1.10 | *Pseudomonas sp.* | 1 | 2 |
| Acetaldehyd-Dehydrogenase, CT-AcDH EC 1.2.1.10 | *Comamonas testosteroni* | 4 | 2 |
| Acetaldehyd-Dehydrogenase, CL-AdhE2 EC 1.2.1.10 | *Clostridium Ijungdahlii* | 7 | 2 |
| Alkohol-Dehydrogenase, HL-ADH EC 1.1.1.1 | Pferdeleber | 10 | 3 |
| Alkohol-Dehydrogenase, TB-ADH EC 1.1.1.2 | *Thermoanaerobacter brockii* | 13 | 3 |
| Alkohol-Dehydrogenase, CL-AdhE2 EC 1.1.1.1 | *Clostridium Ijungdahlii* | 7 | 3 |

**Tabelle 1a: Korrelation der Sequenz-Nummern**

| **Enzym** | **SEQ ID NO: Aminosäuresequenz** | **SEQ ID NO: Wildtyp Nukleotidsequenz** | **SEQ ID NO: Codonoptimiere Nukleotidsequenz** |
|---|---|---|---|
| Vinylacetyl-CoA-Isomerase, CA AbfD | 15 | 16 | 17 |
| Vinylacetyl-CoA-Isomerase, SC ChcB | 18 | 19 | 20 |
| Acetaldehyd-Dehydrogenase, PS DmpF | 1 | 2 | 3 |
| Acetaldehyd-Dehydrogenase, CT-AcDH | 4 | 5 | 6 |
| Acetaldehyd-Dehydrogenase + Alkohol-Dehydrogenase, CL-AdhE2 | 7 | 8 | 9 |
| Alkohol-Dehydrogenase, HL-ADH | 10 | 11 | 12 |
| Alkohol-Dehydrogenase, TB-ADH | 13 | 14 | - |

### Anzucht und Expression unter Standardbedingungen

Für die Expression der für die verschiedenen Enzyme kodierenden Gene wurden der Vektor pET-21a(+) (Novagen) mit einem Ampicillin-Resistenzgen sowie der Expressionsstamm *E. coli* BL21 (DE3) verwendet. Die Anzucht der Stämme erfolgte in LB-Medium mit 100 µg/ml Ampicillin (LBₐₘₚ). Zunächst wurden Vorkulturen mit einem Volumen von 5 ml LBₐₘₚ-Medium über Nacht bei 180 UpM geschüttelt. Aus diesen wurden Hauptkulturen (4 x 200 ml LBₐₘₚ-Medium in 2 L-Kolben) auf eine optische Dichte OD₅₈₀ von 0,1 angeimpft und bei 180 UpM und 37°C geschüttelt. Die Expression der rekombinanten Gene wurde nach Erreichen einer OD₅₈₀ von 0,5 mit unterschiedlichen Mengen an IPTG (Isopropyl-D-1-thiogalactopyranosid) (Tabelle 2) induziert. Anschließend wurden die Kulturen bei 25°C für 5 - 6 h bei 180 UpM geschüttelt. Die Zellen wurden nach Ernte durch Zentrifugation bei -20 °C gelagert. Die Ausbeute an Zellen betrug 2 - 3 g pro 800 ml Kultur.

**Tabelle 2: Übersicht über die Expressionsbedingungen für die einzelnen Enzyme.**

| **Enzym** | **Masse [kDa]** | **IPTG [mM]** | **Expressionsdauer [h]** | **Anzucht** |
|---|---|---|---|---|
| SC-ChcB | 28,2 | 0,2 | 5-6 | aerob |
| CA-AbfD | 54,4 | 0,5 | 5-6 | anaerob |
| PS-DmpF | 32,7 | 0,2 | 5-6 | aerob |
| CT-AcDH | 32 | 0,2 | 5-6 | aerob |
| CL-AdhE2 | 95,6 | 0,2 | 5-6 | aerob |

### Expression unter anaeroben Bedingungen

Die Anzucht der Zellen, die die Isomerase CA-AbfD exprimieren, erfolgte unter weitestgehend anaeroben Bedingungen. Hierzu wurde die 10 ml-Vorkultur bei 37°C und 180 UpM in einem Zentrifugenröhrchen über Nacht geschüttelt. Die folgenden Arbeiten wurden unter Argon in einer Glove Bag durchgeführt. Die 300 ml LBₐₘₚ-Hauptkultur mit 0,4 mM FeCl₃ wurde in einem 500 ml-Kolben mit Schraubverschluss auf eine OD₅₈₀ von 0,1 angeimpft und bei 37°C und 180 UpM bis zu einer OD₅₈₀ von 0,6 - 0,8 geschüttelt. Nach Induktion der Genexpression mit 0,5 mM IPTG wurde für 5 - 6 h bei 37°C und 180 UpM geschüttelt und die Zellen durch Zentrifugation geerntet. Die ca. 0,5 g geernteten Zellen wurden bei -20°C gelagert.

### Herstellung von Zellsuspensionen

Zur Herstellung einer Zellsuspension wurden 2 g Zellen in 1 ml 50 mM Kpi-Puffer, pH 7,0 resuspendiert und bei 30°C im Thermomixer vorinkubiert.

### Zellaufschluss mittels Ultraschall

Für den Zellaufschluss mittels Ultraschall wurde eine 25 %-ige Zellsuspension der Zellen in 50 mM Kpi-Puffer pH 7,5 für zweimal 1,5 min in einem 2 ml-Reaktionsgefäß auf Eis mit Ultraschall behandelt. Nach Zentrifugation für 20 min bei 14000 UpM und 4°C wurde der Rohextrakt abgenommen und für die Umsetzungen verwendet.

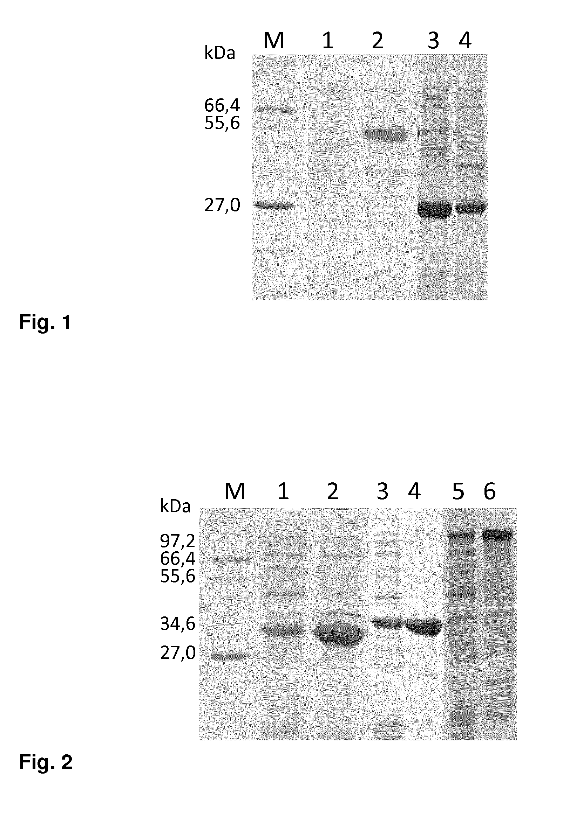

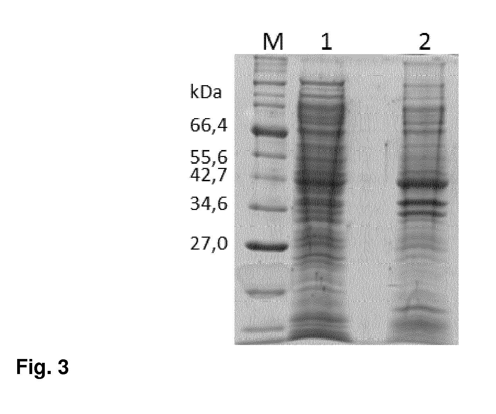

Zur Überprüfung der Expression wurden SDS-Gele angefertigt. Die Figuren 1 bis 3 zeigen SDS-Gele zur Überprüfung der Expression der einzelnen Enzyme. Aufgetragen sind jeweils die lösliche und unlösliche Fraktion. Figur 1 zeigt, dass die Vinylacetyl-CoA-Isomerase aus *Streptomyces collinus* gut exprimiert wurde (Figur 1, Bahnen 3 und 4). Die Vinylacetyl-CoA-Isomerase aus *Clostridium aminobutyricum* wurde zum größeren Teil unlöslich exprimiert (Figur 1, Bahnen 1 und 2). Die Acetaldehyd-Dehydrogenasen aus *Pseudomonas* sp. (Figur 2, Bahnen 1 und 2), *Comamonas testosteroni* (Figur 2, Bahnen 3 und 4) und *Clostridium Ijungdahlii* (Figur 2, Bahnen 5 und 6) wurden teils löslich, teils unlöslich exprimiert. Figur 3 zeigt die Expression der Pferdeleber Alkoholdehydrogenase (Bahnen 1 und 2).

### Beispiel 4: Aktivitätstests der Enzyme und photometrischer Assay

Nach Expression der einzelnen Enzyme wurden diese mit Hilfe der zuvor der Literatur entnommenen photometrischen Assays untersucht. Hierfür wurden die in der Literatur angegebenen Standardsubstrate eingesetzt, die sich, mit Ausnahme von Crotonyl-CoA für die Isomerase-Reaktion, von den in diesem Projekt verwendeten Substraten unterscheiden. Tabelle 3 gibt einen Überblick über die Zusammensetzung der Assays, die Bedingungen sowie die gemessenen Aktivitäten. Das für den jeweiligen Aktivitätstest verwendete Substrat ist in der Tabelle 3 ebenfalls angezeigt.

**Tabelle 3: Übersicht über die photometrischen Assays für die einzelnen Enzyme mit Literaturstellen, die verwendeten Substrate sowie die gemessen Aktivitäten**

| **Schritt** | **Enzym** | **Assay** | **Aktivität (U/ml)** |
|---|---|---|---|
| 1 | Vinylacetyl-CoA-Isomerase CA-AbfD | 35 mM Tris-HCl, pH 7,8 | 0,5 |
| | | 4 mM EDTA | |
| | | 30 µM Crotonyl-CoA | |
| | | 25°C, 266 nm, 1-3 min [1] | |
| 1 | Vinylacetyl-CoA-Isomerase SC-ChcB | 35 mM Tris-HCl, pH 7,8 | 0,5 |
| | | 4 mM EDTA | |
| | | 30 µM Crotonyl-CoA | |
| | | 25°C, 266 nm, 1-3 min [1] | |
| 2 | Acetaldehyd-Dehydrogenase PS-DmpF | 50 mM MES-Puffer, pH 7,8 | 0 (Crotonyl-CoA) |
| | | 0,25 mM NADH | 0,8 (Acetyl-CoA) |
| | | 0,01 µM Substrat | 0,7 (Butyryl-CoA |
| | | 30°C, 340 nm, 1 min [2] | 0,2 (Vinylacetyl-/Crotonyl-CoA) |
| 3 | Alkohol-Dehydrogenase HL-ADH | 50 mM TEA-Puffer, pH 7,0 | 1,8 |
| | | 0,2 mM Crotonaldehyd | |
| | | 0,25 mM NADH | |
| | | 30°C, 340 nm, 1 min | |
| 3 | Alkohol-Dehydrogenase TB-ADH | 100 mM TEA-Puffer, pH 7,8 | 3,8 |
| | | 0,2 mM Crotonaldehyd | |
| | | 0,25 mM NADH | |
| | | 30°C, 340 nm, 1 min | |

| | | | |
|---|---|---|---|
| [1] H.C. Rilling, M.J. Coon, Journal of Biological Chemistry, 1960, 235, 3087. [2] D. Burdette, J.G. Zeikus, Biochem. J, 1994, 302 (Pt1), 163. | | | |

Die Tabelle 3 zeigt, dass für alle im Rahmen dieser Erfindung beschriebenen Reaktionen entsprechende Assays zur Verfügung stehen. Bei allen Enzymen wurde eine Aktivität mit Hilfe der photometrischen Analyse gemessen. Bei der Isomerase-Reaktion (Schritt 1) und der Acetaldehyd-Dehydrogenase-Reaktion (Schritt 2) konnte mit den hier relevanten Substraten Crotonyl-CoA (kommerziell erhältlich bei Sigma) und dem gemäß den Beispielen 1 und 2 synthetisierten Vinylacetyl-CoA (welches im Gemisch mit Crotonyl-CoA vorlag) eine Aktivität photometrisch gemessen werden. Bezüglich der Reaktion gemäß Schritt 3 (Alkohol-Dehydrogenasen) konnten mit den Standardsubstraten wie auch mit ähnlichen Substraten Aktivitäten nachgewiesen werden.

### Inhibierung der Acetaldehyd-Dehydrogenase PS-DmpF durch Crotonyl-CoA

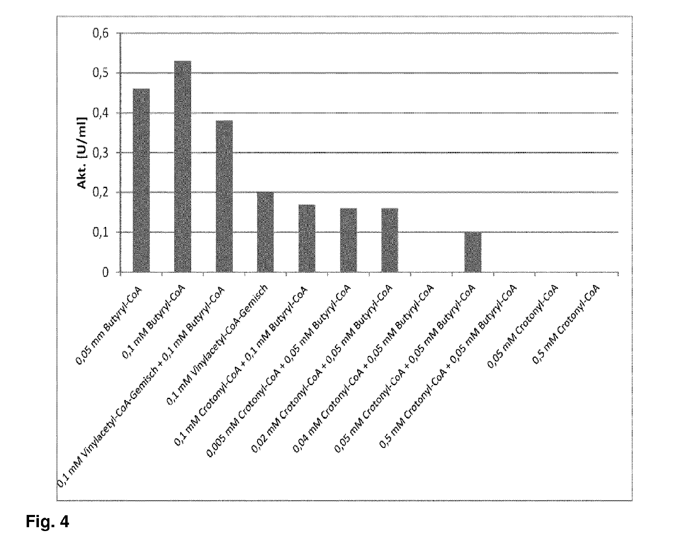

Im Rahmen der Photometertests wurde festgestellt, dass die Acetaldehyd-Dehydrogenase DmpF aus *Pseudomonas sp.* keine Aktivität gegenüber Crotonyl-CoA zeigte. Daher wurde untersucht, ob Crotonyl-CoA kein Substrat für dieses Enzym darstellt oder eine inhibitorische Wirkung auf das Enzym aufweist. Dazu wurden Butyryl-CoA, welches von der Acetaldehyd-Dehydrogenase umgesetzt wird, und Crotonyl-CoA in unterschiedlichen Verhältnissen gemischt und die Aktivität der PS-DmpF gegen diese Substratmischungen untersucht (Figur 4). Die in Figur 4 zusammengefassten Ergebnisse zeigen, dass die Aktivität der PS-DmpF abnahm, je mehr Crotonyl-CoA im Ansatz enthalten ist. Dies bedeutet, dass Crotonyl-CoA inhibierend auf die Acetaldehyd-Dehydrogenase wirkt.

### Beispiel 5: Reaktionen für die Aktivitätstests und Probennahme

### 1. Substrat: Butyryl-CoA

Da für einige Acetaldehyd-Dehydrogenasen die Reduktion von Butyryl-CoA in der Literatur beschrieben ist, wurden die Enzyme mit 2,5 mM Butyryl-CoA umgesetzt. Die Ansätze zur Umsetzung von Butyryl-CoA wurden in einem Volumen von 0,6 ml durchgeführt. Um das für diesen Aktivitätstest gewünschte Produkt (1-Butanol) zu erhalten, wurde die Acetaldehyd-Dehydrogenasen mit der Alkohol-Dehydrogenase aus Pferdeleber (HL-ADH) sowie einer Glukose-Dehydrogenase (GDH) für die Cofaktorregenerierung gekoppelt.

Für die Aktivitätstests wurden 0,6 ml-Ansätze mit folgender Zusammensetzung verwendet:
2,5 bzw. 5 mM Butyryl-CoA,
40% (v/v) Rohextrakt E. *coli* BL21 (DE3), der die entsprechende Acetaldehyd-Dehydrogenase exprimierte,
1 U HL-ADH (Alkoholdehydrogenase-Aktivität gegenüber Standardsubstrat),
50 mM Glucose,
2 mM NAD,
2 U GDH (Glucosedehydrogenase),
25 mM MES-Puffer pH 7,0.

In bestimmten Zeitabständen wurden den Ansätzen Aliquots entnommen und nach einer Extraktion auf das gebildete Produkt 1-Butanol getestet. Die Analyse der Aktivitätstests der Acetaldehyd-Dehydrogenasen erfolgten mittels Gaschromatographie.

### 2. Substrat: Vinylacetyl-CoA/Crotonyl-CoA

Die Ansätze zur Umsetzung von Gemischen enthaltend Vinylacetyl-CoA/Crotonyl-CoA wurden ebenfalls in einem Volumen von 0,6 ml bzw. 1 ml durchgeführt. Die Aktivitätstests der Acetaldehyd-Dehydrogenasen erfolgten mittels Gaschromatographie. Um das gewünschte Produkt Alkohol (Vinylethanol bzw. Crotylalkohol) zu erhalten, wurden die Acetaldehyd-Dehydrogenasen mit der Alkohol-Dehydrogenase aus Pferdeleber (HL-ADH) sowie einer Glukose-Dehydrogenase (GDH) für die Cofaktorregenerierung gekoppelt.

Für die Aktivitätstests wurden 0,6 ml-Ansätze mit folgender Zusammensetzung verwendet:
2,5 bzw. 5 mM Vinylacetyl-CoA/Crotonyl-CoA-Gemisch,
40% (v/v) Rohextrakt E. *coli* BL21 (DE3), der die entsprechende Acetaldehyd-Dehydrogenase exprimierte,
1 U HL-ADH (Aktivität gegenüber Standardsubstrat),
50 mM Glucose,
2 mM NAD,
2 U GDH (Glucosedehydrogenase),
25 mM MES-Puffer pH 7,0.

Weiterhin wurden Ansätze mit ganzen Zellen, die Isomerase exprimieren, durchgeführt. Für die eingesetzten Zellsuspensionen wurden 200 mg Zellen in 1 ml 50 mM MES-Puffer pH 7,0 suspendiert. Die Ansätze mit einem Volumen von 1 ml hatten die folgende Zusammensetzung:
350 µl Zellsuspension *E. coli* BL21 (DE3)/pET-21 a-SC-ChcB bzw. CA-AbfD (ex-primiert Isomerase),
350 µl Rohextrakt *E. coli* BL21 (DE3)/PS-DmpF (exprimierte Acetaldehyd-Dehydrogenase),
1,25 U HL-ADH (Aktivität bezogen auf Standardsubstrat),
2,5 mM Vinylacetyl-CoA/Crotonyl-CoA-Gemisch,
1 U GDH (Glucosedehydrogenase),
50 mM Glucose,
2 mM NAD,
50 mM MES-(2-(N-Morpholino)ethansulfonsäure-)Puffer, pH 7,0.

Parallel wurden bei allen Versuchen Kontrollen durchgeführt, die kein Substrat bzw. anstatt eines Rohextrakt einer Enzym-exprimierenden Zelle eine Leervektorkontrolle (Rohextrakt *E*. *coli* BL21 (DE3)-pET21a(+)), d.h. einen Rohestrakt einer Zelle, die das entsprechende Enzym nicht exprimiert, enthielten.

Die Ansätze wurden in 1,5 ml-Reaktionsgefäßen bei 30°C und 500 UpM in einem Thermomixer gründlich gemischt. Während der Inkubation der Ansätze wurden in bestimmten Zeitintervallen (z.B. 0, 0,5, 1, 2 und 20 h) Proben entnommen. Zur Probennahme wurden je 100 µl Probe mit 200 µl MTBE (Methyl-*tert*-butylether) versetzt, 1 min gründlich gemischt und zur Phasentrennung 5 min bei 14000 UpM zentrifugiert. Die organische Phase wurde abgenommen und gaschromatographisch untersucht.

Die in diesem Beispiel beschriebenen Methoden wurden in den in Beispiel 8 beschriebenen Versuchen angewendet.

### Beispiel 6: Gaschromatographischer Nachweis der Substanzen

Für den gaschromatographischen Nachweis der verschiedenen Substanzen wurden Alkohol-Standards der einzelnen Substanzen in Wasser gelöst und eine Verdünnungsreihe hergestellt. Von der Verdünnung wurden 100 µl mit 200 µM MTBE versetzt, 1 min gründlich gemischt und zur Phasentrennung 5 min bei 14000 UpM zentrifugiert. Die organische Phase wurde abgenommen und gaschromatographisch untersucht.

Vinylacetaldehyd ist nicht kommerziell erhältlich und wurde aus dem korrespondierenden Diethylacetal freigesetzt. Hierzu wurden 10 µl des Diethylacetals mit 500 µl 2 %-iger HCl und 500 µl DMSO für 5 min bei Raumtemperatur gerührt. Dabei wird Vinylacetaldehyd-diethylacetal quantitativ gespalten.

Crotonaldehyd und Vinylacetaldehyd können im Gaschromatographen (GC) nicht unterschieden werden. Der Grund hierfür ist wahrscheinlich, dass bei der hohen Injektortemperatur eine Isomerisierung von Vinylacetaldehyd zu Crotonaldehyd erfolgt. Um die beiden Aldehyde voneinander zu unterscheiden, wurden sie mit NaBH₄ zu den korrespondierenden Alkoholen reduziert, die im Gaschromatographen unterschiedliche Retentionszeiten aufweisen. Dazu wurden 100 µl Aldehyd-Probe mit 2 mg NaBH₄ versetzt und 30 min bei Raumtemperatur inkubiert. Nach Ansäuern mit 50 µl Essigsäure (5 min Inkubation bei Raumtemperatur) wurde mit 150 µl MTBE extrahiert und die organische Phase im Gaschromatographen gemessen.

Die in diesem Beispiel beschriebenen Methoden wurden in den in Beispiel 9 und 10 beschriebenen Versuchen angewendet.

### Beispiel 7: Nachweis der Edukte und Produkte mittels Gaschromatographie

Die in Beispiel 4 eingesetzten Edukte bzw. erhaltenen Produkte wurden mittels Gaschromatographie nachgewiesen. Wie in Beispiel 4 erwähnt, wurden während der Inkubation der Ansätze in bestimmten Zeitintervallen Proben genommen. Zur Probennahme wurden je 100 µl Probe mit 200 µl MTBE (Methyl-*tert-*butylester) versetzt, 1 min gründlich gemischt und zur Phasentrennung 5 min bei 14000 UpM zentrifugiert. Die organische Phase wurde abgenommen und gaschromatographisch untersucht.

Die Analyse erfolgte am Gaschromatographen Focus GC (Thermo). Hierfür wurde die Säule Innopeg-2000 (30 m x 0,25 mm x 0,25 µm, CS Chromatographie Service) verwendet. Als Trägergas wurde Helium eingesetzt. Das Temperaturprogramm für die Analytik der Vinyl- und Crotylverbindungen war wie folgt:

| | | |
|---|---|---|
| | 60°C | 6 min |
| 50°C/min | 220°C | 0,8 min |

Zur Analyse der Butylverbindungen wurde das folgende Temperaturprogramm verwendet:

| | | |
|---|---|---|
| | 40°C | 10 min |
| 30°C/min | 200°C | 0 min |

### Beispiel 8: Aktivitätstests von Acetaldehyd-Dehydrogenasen mit Butyryl-CoA als Substrat

Die Aktivitätstests der Acetaldehyd-Dehydrogenasen erfolgten mittels Gaschromatographie. Um das gewünschte Produkt Alkohol zu erhalten, wurden die Acetaldehyd-Dehydrogenasen mit der Alkohol-Dehydrogenase aus Pferdeleber (HL-ADH) sowie einer Glukose-Dehydrogenase (GDH) für die Cofaktorregenerierung gekoppelt (siehe Beispiel 4). Als Negativkontrolle wurde Rohextrakt aus Zellen eingesetzt, die einen Leervektor anstatt eines Vektors mit dem für die entsprechende Acetaldehyd-Dehydrogenase kodierenden Gens enthielten.

Da für einige Acetaldehyd-Dehydrogenasen die Reduktion von Butyryl-CoA in der Literatur beschrieben ist, wurde für die Aktivitätstests der Enzyme zunächst 2,5 mM Butyryl-CoA eingesetzt. Nach bestimmten Zeiten wurden den Ansätzen Proben entnommen und nach Extraktion auf Bildung von 1-Butanol analysiert. Die Ergebnisse werden in der folgenden Tabelle 4 dargestellt.

**Tabelle 4: Gemessene Konzentrationen an 1-Butanol bei der Reduktion von 2,5 mM Butyryl-CoA mit unterschiedlichen Acetaldehyd-Dehydrogenasen bzw. Negativkontrolle pET-21a**

| **Rohextrakt** | **Probennahme [min]** | **1-Butanol [µM]** |
|---|---|---|
| CT-AcDH | 0 | 0 |
| | 30 | 473 |
| | 60 | 908 |
| | 1200 | 880 |
| CL-AdhE2 | 0 | 0 |
| | 30 | 591 |
| | 60 | 563 |
| | 1200 | 591 |
| PS-DmpF | 0 | 0 |
| | 30 | 594 |
| | 60 | 564 |
| | 1200 | 806 |
| pET-21 a | 0 | 0 |
| | 30 | 0 |
| | 60 | 77 |
| | 1200 | 0 |

Die gaschromatographischen Aktivitätstests mit Butyryl-CoA zeigten, dass Butyryl-CoA von den jeweiligen Enzymen umgesetzt wurde. Daraus konnte gefolgert werden, dass die Enzymtaschen der Acetaldehyd-Dehydrogenasen PS-DmpF, CT-AcDH und CL-AdhE2 ausreichend groß für C4-Bausteine sind.

### Beispiel 9: Aktivitätstests von Acetaldehyd-Dehydrogenasen mit einem Gemisch aus Vinylacetyl-CoA und Crotonyl-CoA als Substrat

Das gemäß Beispiel 2 hergestellte Vinylacetyl-CoA/Crotonyl-CoA-Gemisch wurden in einem Vorversuch jeweils chemisch mit NaBH₄ reduziert, um die Zusammensetzung zu überprüfen. Es zeigte sich, dass nach Reduktion einer Probe dieses Gemisches Vinylethanol und Crotylalkohol im Verhältnis 30:70 (oder 50:50 je nach Charge) erhalten wurden.

Die Reaktionsansätze enthielten neben der jeweiligen Acetaldehyd-Dehydrogenase 2,5 mM des Vinylacetyl-CoA/Crotonyl-CoA-Gemischs. Die Enzyme CT-AcDH und CL-AdhE2 reduzierten das Vinylacetyl-CoA/Crotonyl-CoA-Gemisch zu Vinylethanol und Crotylalkohol (Tabelle 5). Die Leervektorkontrolle zeigte keine Aktivität.

**Tabelle 5: Gemessene Konzentrationen an Vinylethanol und Crotylalkohol bei der Reduktion von 2,5 mM des Vinylacetyl-CoA/Crotonyl-CoA-Gemisches (Verhältnis Vinyl-/Crotylverbindung 30:70) mit unterschiedlichen Acetaldehyd-Dehydrogenasen bzw. Negativkontrolle pET-21 a (1. Versuch)**

| **Rohextrakt** | **Probennahme [min]** | **Vinylethanol [µM]** | **Crotylethanol [µM]** |
|---|---|---|---|
| CT-AcDH | 0 | 0 | 0 |
| | 30 | 46 | 57 |
| | 60 | 53 | 68 |
| | 1200 | 60 | 24 |
| CL-AdhE2 | 0 | 26 | 0 |
| | 30 | 26 | 39 |
| | 60 | 26 | 36 |
| | 1200 | 33 | 45 |
| pET-21 a | 0 | 0 | 0 |
| | 30 | 0 | 0 |
| | 60 | 0 | 0 |
| | 1200 | 0 | 0 |
| PS-DmpF | 0 | 0 | 0 |
| | 30 | 0 | 0 |
| | 60 | 7 | 0 |
| | 1200 | 0 | 0 |

Der Versuch wurde erneut durchgeführt. Auch hier zeigte sich, dass die beiden Enzyme CT-AcDH und CL-AdhE2 aktiv waren. In diesem Versuch wurde das Vinylacetyl-CoA/Crotonyl-CoA-Gemisch von der CT-AcDH nur zu Vinylethanol und von der CL-AdhE2 hauptsächlich zu Vinylethanol reduziert; hier wurde in einer Probe auch ein Crotylalkohol-Peak analysiert. Die Ergebnisse des zweiten Versuchs wurden in Tabelle 6 zusammengefasst.

**Tabelle 6: Gemessene Konzentrationen an Vinylethanol und Crotylalkohol bei der Reduktion von 2,5 mM des Vinylacetyl-CoA/Crotonyl-CoA-Gemisches (Verhältnis Vinyl-/Crotylverbindung 30:70) mit unterschiedlichen Acetaldehyd-Dehydrogenasen bzw. Negativkontrolle pET-21 a (2. Versuch)**

| **Rohextrakt** | **Probennahme [min]** | **Vinylethanol [µM]** | **Crotylethanol [µM]** |
|---|---|---|---|
| CT-AcDH | 0 | 0 | 0 |
| | 30 | 26 | 0 |
| | 60 | 30 | 0 |
| | 1200 | 42 | 0 |
| CL-AdhE2 | 0 | 0 | 0 |
| | 30 | 10 | 0 |
| | 60 | 15 | 0 |
| | 1200 | 18 | 18 |
| pET-21a | 0 | 0 | 0 |
| | 30 | 0 | 0 |
| | 60 | 0 | 0 |
| | 1200 | 0 | 0 |
| PS-DmpF | 0 | 0 | 0 |
| | 30 | 0 | 0 |
| | 60 | 0 | 0 |
| | 1200 | 0 | 0 |

In der folgenden Tabelle 7 werden die Ergebnisse der enzymatischen Reduktionen mit den aktiven Enzymen sowie der chemischen Reduktionen des Vinylacetyl-CoA/Crotonyl-CoA-Gemisches zusammengefasst. Die Zusammenfassung zeigt, dass die Ergebnisse der chemischen und enzymatischen Reduktion übereinstimmen.

**Tabelle 7:**

| | | | |
|---|---|---|---|
| **Vinylacetyl**-**CoA / Crotonyl-CoA-Gemisch** | **Reduktion mit** | **Produkte** | |
| | | **Vinylethanol** | **Crotylethanol** |
| | CT-AcDH | + | (+) |
| | CL-AdhE2 | + | (+) |
| | NaBH₄ | + | + |

Die vorliegenden Ergebnisse zeigen, dass zwei der untersuchten Acetaldehyd-Dehydrogenasen Aktivität gegenüber Vinylacetyl-CoA, welches synthesebedingt stets im Gemisch mit Crotonyl-CoA vorlag, ohne Inhibition durch Crotonyl-CoA unter den gewählten Bedingungen aufwiesen: die Acetaldehyd-Dehydrogenase CT-AcDH aus *Comamonas testosteroni* (*C. testosteroni*) und die Acetaldehyd-Dehydrogenase CL-AdhE2 aus *C. Ijungdahlii.* Dabei wies die Acetaldehyd-Dehydrogenase aus *C. testosteroni* Selektivität gegenüber Vinylacetyl-CoA auf.

Reines Crotonyl-CoA wurde von der Acetaldehyd-Dehydrogenase aus *Pseudomonas sp.* kaum oder gar nicht umgesetzt. Dies deutete darauf hin, dass Crotonyl-CoA eine mögliche inhibierende Wirkung auf das Enzym hat sowie auf eine Selektivität dieses Enzyms für Vinylacetyl-CoA.

### Beispiel 10: Untersuchungen zur Cofaktorspezifität der Aldehyd-Dehydrogenasen

Um die Cofaktorspezifität der Aldehyd-Dehydrogenasen CT-AcDH und CL-AdhE2 zu untersuchen, wurden beide Enzyme mit dem Vinylacetyl-CoA/Crotonyl-CoA-Gemisch zusammen mit NAD bzw. NADP inkubiert. Es zeigte sich, dass die CT-AcDH sowohl NAD als auch NADP als Cofaktor akzeptiert, während die CL-AdhE2 NAD-abhängig ist (Tabelle 8).

**Tabelle 8: Zusammenfassung der Aktivitätstests der CT-AcDH bzw. der CL-AdhE2 Acetaldehyd-Dehydrogenasen in Anwesenheit von NAD bzw. NADP und 2,5 mM bzw. 5 mM Vinylacetyl-CoA/Crotonyl-CoA-Gemisch**

| **Rohextrakt** | **Vinyl-acetyl-CoA / Crotonyl-CoA [mM]** | **Cofaktor** | **Probennahme [min]** | **Vinylethanol [µM]** | **Crotylethanol [µM]** |
|---|---|---|---|---|---|
| CT-AcDH | 2,5 | NAD | 0 | 0 | 0 |
| | | | 30 | 24 | 0 |
| | | | 60 | 29 | 0 |
| | | | 1200 | 42 | 0 |
| | | NADP | 0 | 0 | 0 |
| | | | 30 | 18 | 0 |
| | | | 60 | 23 | 0 |
| | | | 1200 | 46 | 29 |
| | 5 | NAD | 0 | 0 | 0 |
| | | | 30 | 15 | 0 |
| | | | 60 | 40 | 0 |
| | | | 1200 | 34 | 0 |
| | | NADP | 0 | 0 | 0 |
| | | | 30 | 11 | 0 |
| | | | 60 | 18 | 0 |
| | | | 1200 | 47 | 0 |
| CL-AdhE2 | 2,5 | NAD | 0 | 10 | 0 |
| | | | 30 | 42 | 25 |
| | | | 60 | 40 | 31 |
| | | | 1200 | 52 | 50 |
| | | NADP | 0 | 0 | 0 |
| | | | 30 | 0 | 0 |
| | | | 60 | 0 | 0 |
| | | | 1200 | 0 | 0 |
| | 5 | NAD | 0 | 8 | 0 |
| | | | 30 | 47 | 25 |
| | | | 60 | 54 | 16 |
| | | | 1200 | 31 | 46 |
| | | NADP | 0 | 0 | 0 |
| | | | 30 | 0 | 0 |
| | | | 60 | 0 | 0 |
| | | | 1200 | 0 | 0 |

### Beispiel 11: Versuche mit der bifunktionalen Aldehyd-/Alkohol-Dehydrogenase CL-AdhE2

In einem weiteren Versuch wurden die beiden Acetaldehyd-Dehydrogenasen CT-AcDH und CL-AdhE2 ohne Alkohol-Dehydrogenase mit dem aus Beispiel 2 erhaltenen Vinylacetyl-CoA/Crotonyl-CoA-Gemisch eingesetzt. Im Kontrollversuch mit einer separaten Alkohol-Dehydrogenase (+ HL-ADH) konnten mit beiden Enzymen Vinylethanol und Crotylalkohol detektiert werden. Bei Abwesenheit einer separaten Alkohol-Dehydrogenase (- HL-ADH) wurden im Ansatz mit der Acetaldehyd-Dehydrogenase CT-AcDH keine Alkohole gebildet. Der Ansatz mit der Acetaldehyd-Dehydrogenase CL-AdhE2-Ansatz zeigte dennoch die Bildung der beiden Alkohole (Tabelle 9). Der Grund hierfür ist, dass es sich bei dem Enzym CL-AdhE2 im Gegensatz zur CT-AcDH um eine bifunktionale Aldehyd-/Alkohol-Dehydrogenase handelt, so dass dieses Enzym in der Lage war, beide Reduktionsschritte zu katalysieren. Daher wurde Vinylethanol erhalten, ohne dass eine zusätzliche Alkoholdehydrogenase eingesetzt werden musste.

**Tabelle 9: Gemessene Konzentrationen an Vinylethanol und Crotylalkohol bei der Reduktion von 2,5 mM des Vinylacetyl-CoA/Crotonyl-CoA-Gemisches mit und ohne Zusatz von der Alkoholdehydrogenase HL-ADH**

| **Enzyme** | | **Probennahme [min]** | **Vinylethanol [µM]** | **Crotylethanol [µM]** |
|---|---|---|---|---|
| **AcDH** | **ADH** | | | |
| CT-AcDH | + HL-ADH | 0 | 22 | 0 |
| | | 30 | 33 | 45 |
| | | 60 | 41 | 63 |
| | | 1320 | 75 | 95 |
| | - HL-ADH | 0 | 0 | 0 |
| | | 30 | 0 | 0 |
| | | 60 | 0 | 0 |
| | | 1320 | 0 | 0 |
| CL-AdhE2 | + HL-ADH | 0 | 0 | 0 |
| | | 30 | 23 | 27 |
| | | 60 | 20 | 56 |
| | | 1320 | 0 | 0 |
| | - HL-ADH | 0 | 0 | 0 |
| | | 30 | 8 | 55 |
| | | 60 | 34 | 39 |
| | | 1320 | 0 | 0 |

### Beispiel 12: Aktivitätstests mit Vinylacetyl-/Crotonyl-CoA als Substrat

Vinylacetyl-/Crotonyl-CoA wurde mit den Enzymen Acetaldehyd-Dehydrogenase aus *C. testosteroni* CT-AcDH (Rohextrakt), in Anwesenheit der Alkohol-Dehydrogenase aus Pferdeleber HL-ADH, NAD sowie Glucose-Dehydrogenase GDH zur Cofaktorregenierung inkubiert. Als Negativkontrolle wurde Substrat mit Leervektor-Rohextrakt eingesetzt, als Positivkontrolle enthielt einer der Ansätze Vinylethanol. Die Ansätze hatten folgende Zusammensetzung:
2,5 mM Vinylacetyl-CoA/Crotonyl-CoA-Gemisch,
1 U HL-ADH (Aktivität gegenüber Standardsubstrat),
50 mM Glucose,
1 mM NAD,
2 U GDH (Glucosedehydrogenase),
25 mM MES-Puffer pH 7,0
und
1) 40% (v/v) Rohextrakt *E*. *coli* BL21 (DE3) (Leervektor-Rohextrakt) oder
2) 40% (v/v) Rohextrakt *E*. *coli* BL21 (DE3), der die Acetaldehyd-Dehydrogenase CT-AcDH exprimierte oder
3) 40% (v/v) Rohextrakt *E. coli* BL21 (DE3) (Leervektor-Rohextrakt), 2 mM Vinylethanol.

Die Ansätze wurden bei Raumtemperatur in Rollrandgläsern gerührt. Zum Start der Reaktion sowie nach 2 h Reaktionszeit wurden Proben entnommen und entweder nach MTBE Extraktion gaschromatographisch untersucht (siehe Beispiele 6 und 7) oder sterilfiltriert (siehe nächster Abschnitt "HPLC/MS nach Derivatisierung"). Die Ergebnisse werden in Tabelle 10 zusammengefasst.

**Tabelle 10: Ergebnisse der enzymatischen Umsetzung von 2,5 mM Vinylacetyl-/Crotonyl-CoA (2) sowie Negativ- (1) und Positivkontrolle (3).**

| **Ansatz** | **Enzyme** | **Probennahme [min]** | **Fläche Vinylethanol** | **Fläche Crotylalkohol** |
|---|---|---|---|---|
| 1 | - | 0 | 0 | 0 |
| | | 120 | 0 | 0 |
| 2 | CT-AcDH, HL-ADH | 0 | 0 | 0 |
| | | 120 | 588 | 381 |
| 3 | - | 0 | 75127 | 0 |
| | | 120 | 69634 | 0 |

Die Versuche zeigten, dass beide CoA-Verbindungen von der CT-AcDH reduziert wurden. Basierend auf der Peakfläche der Positivkontrolle wurden die Konzentrationen der Alkohole in der Probe 2 berechnet (siehe Tabelle 11).

### HPLC/MS nach Derivatisierung

Zur weiteren Untersuchung mittels HPLC/MS nach Derivatierung wurden 200 µl wässriger, sterilfiltrierter Probenlösung mit 2,0 mL einer Lösung von Phenylisocyanat (im Überschuss) in Acetonitril versetzt. Im geschlossenen Gefäß wurde die Lösung auf 50° C erwärmt und über 16 Stunden inkubiert. Der Alkohol reagierte mit Phenylisocyanat zum entsprechenden Carbamat. Nach Zugabe von 1,0 mL Methanol wurde die Lösung 10 min geschüttelt. Unlösliche Bestandteile der Probenlösung wurden mit einem Spritzenvorsatzfilter abfiltriert. Das Filtrat wurde mittels HPLC an einer RP-Phase chromatographisch getrennt und mit einem Massendetektor identifiziert und quantifiziert (AB Sciex API3200, MRM-Modus). Die Elutionszeiten der Isomeren Vinylethanol bzw. Crotylalkohol wurden durch Kalibrierungen mit Reinsubstanzen ermittelt. Beide Isomere zeigten getrennte und eindeutige Peaks. Die Berechnung der Konzentrationen erfolgte über Kalibrierungen mit unterschiedlichen Konzentrationen an Vinylethanol bzw. Crotylalkohol.

Elutionszeiten HPLC: 4,9 min Carbamat aus Methanol und Phenylisocyanat, 7,2 min Carbamat aus Vinylethanol und Phenylisocyanat, 7,4 min Carbamat aus Crotylalkohol und Phenylisocyanat.

M(Phenylisocyanat + Crotylalkohol/Vinylethanol + H+) = 192 g/Mol

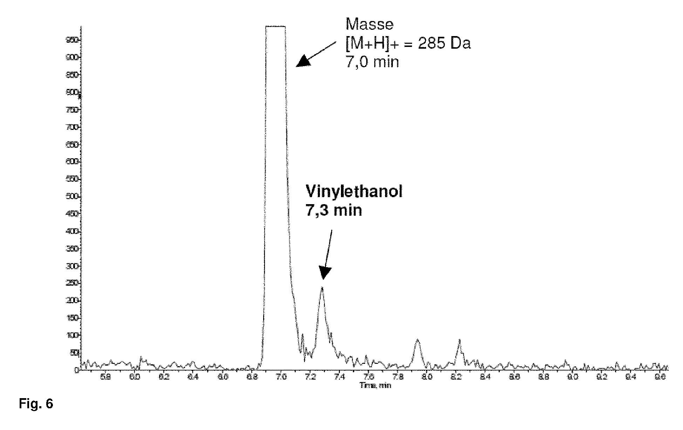

In der Methode reagierte eine weitere Substanz ebenfalls mit dem Derivatisierungsmittel und eluierte kurz vor dem Vinylethanol (Figur 6). Die MS-Analyse zeigte eine Masse [M+H]+ von 285 Da. Im Verhältnis zur Größe des Alkoholpeaks war der Peak dieser Substanz so groß, dass er im MS ein Artefakt erzeugte. Da in der Dotierung der Alkohol wiedergefunden wurde und die Elutionszeit nicht von der unbekannten Komponente beeinflusst wurde, ist die Methode dennoch gut zum Nachweis von Vinylethanol geeignet.

**Tab. 11: Ergebnisse der enzymatischen Umsetzung von 2,5 mM Vinylacetyl-/Crotonyl-CoA.**

| **Rohextrakt** | **Probennahme [min]** | **Konzentration Vinylethanol [µM]** | **Konzentration Crotylalkohol [µM]** |
|---|---|---|---|
| Acetaldehyd-Dehydrogenase aus *C. testosteroni* CT-AcDH | 0 | 0 | 0 |
| | | 0 | 0 |
| | 120 | 17 | 11 |
| | | 20 | n.d. |
| Acetaldehyd-Dehydrogenase aus *C. testosteroni* CT-AcDH | 0 | 0 | 0 |
| | 30 | 24 | 0 |
| | 60 | 29 | 0 |
| | 1200 | 42 | 0 |

## Patentansprüche

1. Ein Verfahren zur Umsetzung einer Verbindung mit der Formel (II) [Vinylacetyl-CoA, 3-Butenoyl-CoA] 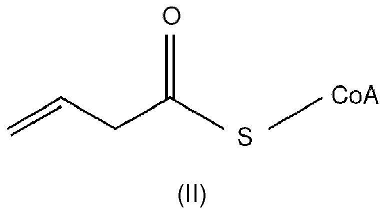 durch Kontaktieren der Verbindung mit der Formel (II) mit einer Aldehyd-Dehydrogenase-Aktivität, wobei eine Verbindung mit der Formel (III) [Vinylacetaldehyd, 3-Butenal] erhalten wird

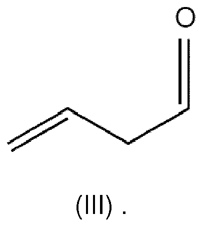

2. Das Verfahren gemäß Anspruch 1, **dadurch gekennzeichnet, dass** die Verbindung mit der Formel (III) weiter umgesetzt wird zu einer Verbindung mit der Formel (IV) [Vinylethanol, 3-Buten-1-ol] 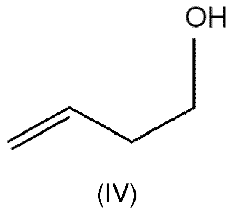 durch Kontaktieren der Verbindung mit der Formel (III) mit einer Alkohol-Dehydrogenase-Aktivität.

3. Das Verfahren gemäß Anspruch 1 oder 2, **dadurch gekennzeichnet, dass** die Verbindung mit der Formel (II) erhalten wird durch Umsetzung einer Verbindung mit der Formel (I) [Crotonyl-CoA] 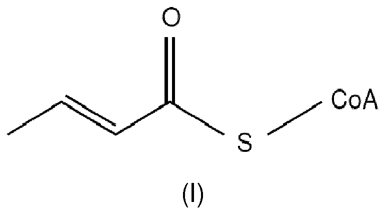 durch Kontaktieren mit einer Vinylacetyl-CoA-Isomerase-Aktivität.

4. Das Verfahren gemäß irgendeinem der Ansprüche 1 bis 3, **dadurch gekennzeichnet, dass** die Aldehyd-Dehydrogenase-Aktivität eine acylierende Aldehyd-Dehydrogenase-Aktivität ist.

5. Das Verfahren gemäß irgendeinem der Ansprüche 1 bis 4, **dadurch gekennzeichnet, dass** die Aldehyd-Dehydrogenase-Aktivität von einer acylierenden Acetaldehyd-Dehydrogenase ausgeht.

6. Das Verfahren gemäß irgendeinem der Ansprüche 1 bis 5, **dadurch gekennzeichnet, dass** die Aldehyd-Dehydrogenase-Aktivität von einer Aldehyd-Dehydrogenase ausgeht, die ausgewählt ist aus der Gruppe bestehend aus *Pseudomonas sp.* DmpF (SEQ ID NO: 1), *Comamonas testosteroni* AcDH (SEQ ID NO: 4) und *Clostridium Ijungdahlii* Adh E2 (SEQ ID NO: 7).

7. Das Verfahren gemäß Anspruch 6, **dadurch gekennzeichnet, dass** die Aldehyd-Dehydrogenase eine in irgendeinem der SEQ ID NOs: 1, 4 und 7 gezeigte Aminosäuresequenz aufweist oder eine Aminosäuresequenz aufweist, deren Aminosäurepositionen mindestens zu 15 % identisch sind mit irgendeinem der in SEQ ID NOs: 1, 4 und 7 gezeigten Aminosäuresequenzen.

8. Das Verfahren gemäß irgend einem der Ansprüche 1 bis 7, **dadurch gekennzeichnet, dass** weiterhin eine Alkohol-Dehydrogenase-Aktivität anwesend ist, die von einer Alkohol-Dehydrogenase ausgeht und vorzugsweise ausgewählt ist aus der Gruppe bestehend aus Pferdeleber-Alkoholdehydrogenase (SEQ ID NO: 10), *Thermoanaerobacter brockii* ADH (SEQ ID NO: 13) und *Clostridium Ijungdahlii* AdhE2 (SEQ ID NO: 7).

9. Das Verfahren gemäß irgend einem der Ansprüche 1 bis 8, **dadurch gekennzeichnet, dass** weiterhin eine Vinylacetyl-CoA-Isomerase-Aktivität anwesend ist, die von einer Vinylacetyl-CoA-Isomerase ausgeht und vorzugsweise ausgewählt ist aus der Gruppe bestehend aus *Clostridium aminobutyricum* AbfD SEQ ID NO: 15) und *Streptomyces collinus* ChcB (SEQ ID NO: 18).

10. Das Verfahren gemäß irgendeinem der Ansprüche 1 bis 9, **dadurch gekennzeichnet, dass** das Verfahren in Anwesenheit eines Mikroorganismus durchgeführt wird, der Aldehyd-Dehydrogenase-Aktivität und optional Alkohol-Dehydrogenase-Aktivität und optional Vinylacetyl-CoA-Isomerase-Aktivität besitzt.

11. Ein Verfahren zur enzymatischen Herstellung einer Verbindung mit der Formel (IV) [Vinylethanol, 3-Buten-1-ol] 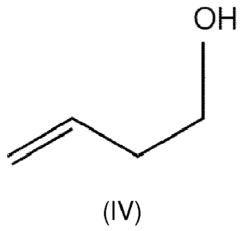 enthaltend die Schritte:
(a) Umsetzung einer Verbindung mit der Formel (II) [Vinylacetyl-CoA, 3-Butenoyl-CoA] 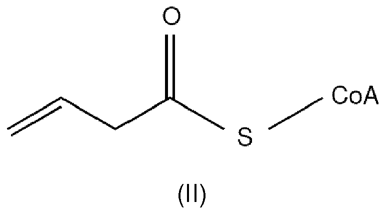 in Anwesenheit einer Aldehyd-Dehydrogenase-Aktivität, wobei eine Verbindung mit der Formel (III) [Vinylacetaldehyd, 3-Butenal] erhalten wird. 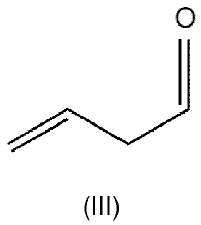
(b) Umsetzung der aus (a) erhaltenen Verbindung mit der Formel (III) in Anwesenheit einer Alkohol-Dehydrogenase-Aktivität wobei die Verbindung mit der Formel (IV) [Vinylethanol, 3-Buten-1-ol] erhalten wird

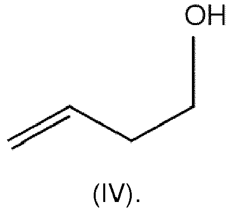

12. Das Verfahren nach Anspruch 11, **dadurch gekennzeichnet, dass** die Verbindung mit der Formel (II) erhalten wird durch Umsetzung einer Verbindung mit der Formel (I) [Crotonyl-CoA] 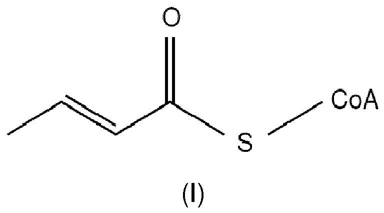 durch Kontaktieren mit einer Vinylacetyl-CoA-Isomerase-Aktivität.

13. Ein Mikroorganismus, **gekennzeichnet durch** die folgenden Merkmale:
a) der Mikroorganismus exprimiert eine Aldehyd-Dehydrogenase, insbesondere eine acylierende Aldehyd-Dehydrogenase, vorzugsweise ausgewählt aus der Gruppe bestehend aus *Pseudomonas sp.* DmpF (SEQ ID NO: 1), *Comamonas testosteroni* AcDH (SEQ ID NO: 4) und *Clostridium Ijungdahlii* AdhE2 (SEQ ID NO: 7), die in der Lage ist, eine Verbindung mit der Formel (III) 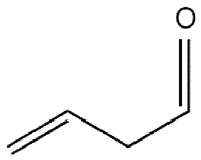 zu erzeugen;
b) optional, der Mikroorganismus exprimiert eine Alkohol-Dehydrogenase, die in der Lage ist, eine Verbindung mit der Formel (IV) 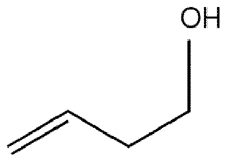 zu erzeugen;
c) optional, der Mikroorganismus exprimiert eine Vinylacetyl-CoA-Isomerase, die in der Lage ist, eine Verbindung mit der Formel (II) 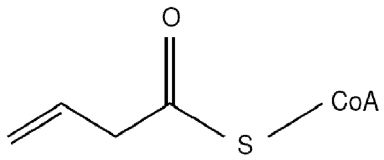 zu erzeugen;
d) der Mikroorganismus ist in der Lage, eine Verbindung mit der Formel (III) 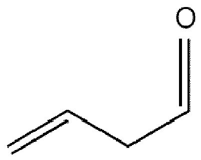 und/oder (IV) 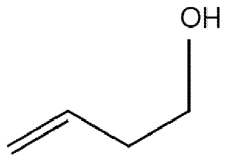 zu erzeugen.

14. Eine Zusammensetzung enthaltend einen Mikroorganismus gemäß Anspruch 13.

15. Die Zusammensetzung gemäß Anspruch 14, enthaltend eine Verbindung mit der Formel (III) und/oder (IV).

16. Verwendung des Mikroorganismus gemäß Anspruch 13 oder der Zusammensetzung gemäß Anspruch 14 zur Herstellung einer Verbindung mit der Formel (III) und/oder (IV).

17. Verfahren zur fermentativen Herstellung einer Verbindung mit der Formel (III) 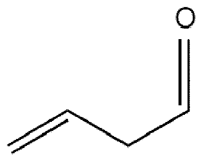 und/oder (IV) 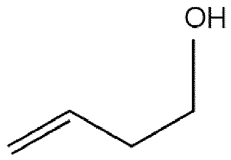 enthaltend die Schritte:
a) Kultivieren des Mikroorganismus gemäß Anspruch 13 in einem geeigneten Medium, wobei eine Fermentationsbrühe erhalten wird, und
b) Anreichern der Verbindung mit der Formel (III) und/oder (IV) in der Fermentationsbrühe aus a).
